# FASCICULE DE BREVET EUROPEEN

(11) **EP 3 448 589 B1**
(45) Date de publication et mention de la délivrance du brevet: **28.10.2020**
(21) Numéro de dépôt: 17725307.7
(22) Date de dépôt: 24.04.2017
(51) Int. Cl.: B07C 5/342, B65B 25/04, B65B 35/58, B65B 57/00

(54) **PROCÉDÉ ET DISPOSITIF D'ORIENTATION D'UN FRUIT OMBILIQUÉ NOTAMMENT EN VUE DE SON EMBALLAGE**
VERFAHREN UND VORRICHTUNG ZUM ORIENTIEREN VON EINEM OBST MIT EINEM UMBILICUS INSBESONDERE ZU SEINEM VERPACKEN
METHOD AND APPARATUS FOR ORIENTING A FRUIT WITH AN UMBILICUS IN PARTICULAR FOR THE PACKAGING THEREOF

(30) Priorité: 28.04.2016 FR 1653810
(43) Date de publication de la demande: 06.03.2019
(73) Titulaire: MAF Agrobotic, 82000 Montauban (FR)
(72) Inventeur: BLANC, Philippe, 82000 Montauban (FR)
(74) Mandataire: Cabinet BARRE LAFORGUE & associés
(86) Numéro de dépôt international: PCT/FR2017/050970
(87) Numéro de publication internationale: WO 2017/187076

(56) Documents cités:
- EP-A1- 0 332 477
- EP-A1- 0 436 244
- EP-A1- 0 727 355
- EP-B1- 1 183 197
- US-A1- 2015 259 088
- US-B1- 6 271 520

## Description

L'invention concerne un procédé d'orientation d'un fruit ombiliqué. Elle s'étend en particulier à un procédé d'emballage de fruits ombiliqués comprenant un procédé d'orientation selon l'invention, à un programme d'ordinateur adapté pour la mise en œuvre d'un procédé d'orientation selon l'invention et/ou d'un procédé d'emballage selon l'invention, à un dispositif d'orientation d'un fruit ombiliqué comprenant une unité de traitement programmable programmée pour la mise en œuvre d'un procédé d'orientation selon l'invention et à un dispositif d'emballage de fruits ombiliqués comprenant au moins un robot de manutention de fruits, ce dispositif d'emballage étant adapté pour la mise en œuvre d'un procédé d'emballage selon l'invention.

Certains fruits dits ombiliqués (pommes, coings, pêches, abricots, certaines tomates, certaines poires,...) comportent à leur périphérie au moins une dépression ombilicale. Il peut s'agir par exemple d'une cuvette oculaire au niveau de l'œil du fruit et/ou d'une cavité pédonculaire. Lors de l'emballage de tels fruits ombiliqués, il est souvent souhaitable de pouvoir contrôler l'orientation dans l'espace du fruit par rapport à l'emballage, pour favoriser sa présentation aux consommateurs. Tel est le cas en particulier lorsque les fruits sont emballés en cagettes ou plateaux, notamment en cagettes ou plateaux alvéolés, chaque alvéole contenant un fruit. Il est en effet alors préférable que tous les fruits d'un même emballage soient orientés avec leur pédoncule dans la même direction et dans le même sens. Il est aussi souhaitable que les fruits soient orientés de façon à présenter vers le haut des portions de surface dont la couleur est la plus attractive possible et qui soit la plus uniforme possible pour un même emballage.

Jusqu'à maintenant, malgré l'automatisation de plus en plus poussée des unités d'emballage modernes, et des recherches anciennes et poussées dans ce domaine, l'orientation des fruits est encore réalisée manuellement par des opérateurs, dont les tâches sont particulièrement pénibles et répétitives.

EP332477 décrit un dispositif de convoyage de fruits ou légumes comprenant deux cylindres longitudinaux entraînés en rotation et un système de progression disposé entre les deux cylindres comprenant une pluralité d'éléments de révolution tels que des boules, se déplaçant en translation entre les cylindres pour pousser les objets parallèlement aux axes des cylindres alors que les objets sont entraînés en rotation. Chaque boule est également animée d'un mouvement de rotation selon un axe perpendiculaire à l'axe de rotation du fruit imparti par la rotation des cylindres jusqu'à ce que la boule vienne se loger dans l'une des deux cavités naturelles des pôles du fruit. Il s'est avéré cependant que ce dispositif n'est pas assez efficace, de nombreux fruits n'étant finalement pas correctement alignés.

EP 0727355 décrit un procédé d'emballage de fruits tels que des pommes et des tomates dans lequel chaque fruit peut être entraîné en rotation par des rouleaux autour d'un premier axe horizontal parallèle à l'axe des rouleaux et autour d'un deuxième axe vertical en vue d'orienter le fruit selon une orientation prédéterminée. Une analyse optique de chaque fruit est réalisée et les images produites sont comparées avec une image de référence pour déterminer que le fruit a atteint une orientation acceptable. Un tel procédé par comparaison avec une image de référence manque de fiabilité, une telle image de référence ne permettant pas en pratique de garantir l'orientation de chaque fruit, compte tenu de la variation importante de formes et de dimensions des fruits par rapport à l'image de référence. Ainsi, ce procédé ne converge pas nécessairement vers une orientation optimale.

EP 1183197 décrit un procédé d'orientation de fruits ombiliqués tels que des pommes ou des poires permettant de les orienter dans la même direction et avec l'inflorescence orientée vers le haut dans des emballages alvéolés. Chaque fruit supporté entre deux rouleaux peut être entraîné en rotation propre autour d'un axe parallèle à l'axe des rouleaux, et peut être soulevé et entraîné en rotation propre autour d'un axe vertical grâce à un support d'élévation rotatif s'insérant entre les rouleaux. Ce document ne décrit pas le procédé pouvant être mis en œuvre pour détecter la position de l'inflorescence et orienter l'inflorescence vers le haut, et suppose que cela est toujours possible par rotation des rouleaux. Or, tel n'est bien évidemment pas le cas, de sorte que ce procédé souffre d'un tel manque de fiabilité qu'il ne peut pas être mis en œuvre en pratique.

WO 2014/068418 décrit un procédé d'orientation d'un fruit ombiliqué dans lequel on entraîne le fruit en rotation propre par des rouleaux autour d'un premier axe X de rotation tout en réalisant des images du profil du fruit à l'aide d'un profilomètre dans un plan d'orientation A comprenant le premier axe X de façon à détecter une cuspide dans le profil, et à mesurer l'angle d'inclinaison de l'axe principal S du fruit par rapport à ce premier axe X. On entraîne ensuite le fruit en rotation autour d'un second axe Y de rotation perpendiculaire au premier axe X de rotation de façon à orienter l'axe principal S du fruit parallèle au premier axe X de rotation dans le plan d'orientation A.

Ce procédé présente aussi plusieurs inconvénients. Tout d'abord, la détection du profil nécessite un profilomètre composé de plusieurs sources de lumière et de plusieurs caméras disposées autour du fruit selon un plan horizontal, ce qui est particulièrement complexe, nécessite de placer des organes fragiles et sensibles au-dessus des rouleaux à proximité immédiate des fruits, au risque de détériorer ses composants fragiles lors des étapes de chargement ou de déchargement des rouleaux. Par ailleurs, les algorithmes d'analyse et de détection de cupsides dans un profil sont à la fois particulièrement complexes et de fiabilité insuffisante. En effet, la détection de l'axe principal par un algorithme de reconnaissance de profil par comparaison avec un profil préétabli ne fournit pas des résultats fiables, le profil préétabli ne correspondant pas nécessairement à celui de tous les objets pouvant être traités, dont le profil est par nature extrêmement variable d'un fruit à l'autre. Il est à noter également que l'efficacité de la détection de l'axe principal du fruit et de sa présence dans un plan horizontal varie considérablement en fonction de l'orientation initiale de cet axe principal par rapport à l'axe de rotation horizontal. En effet, par exemple, si l'axe principal de l'objet est initialement peu incliné par rapport à l'axe de rotation, les variations de profil induites au cours de la rotation sont faibles. Il en résulte, par construction géométrique, une grande marge d'erreur de détection de l'axe principal dans le plan horizontal. Ce procédé ne permet pas non plus d'orienter des fruits exempts de pédoncules. EP 0 436 244 concerne un mécanisme d'orientation pour orienter les fruits,par exemple.

Ainsi, malgré un besoin ressenti de longue date et les différents efforts entrepris antérieurement, aucun procédé d'orientation proposé antérieurement n'est suffisamment fiable pour pouvoir être exploité à l'échelle industrielle en pratique, de sorte que dans les lignes d'emballage les fruits sont encore orientés manuellement.

L'invention vise donc à pallier ces inconvénients.

L'invention vise donc à proposer un procédé d'orientation d'un fruit ombiliqué qui présente une efficacité et une fiabilité accrues, moyennant un faible coût de fabrication, d'installation et d'utilisation.

Elle vise en particulier à proposer un tel procédé d'orientation qui soit suffisamment fiable et efficace, et suffisamment économique pour être compétitif par rapport à des opérations d'orientation manuelle des fruits.

Elle vise également à proposer un tel procédé d'orientation qui soit compatible avec sa mise en œuvre dans un environnement agricole, notamment en association avec une unité d'emballage de fruits à haute cadence, en particulier équipée de robots de manutention.

L'invention vise également à proposer un tel procédé qui permet d'orienter les fruits d'une part avec chaque ombilic orienté selon une direction prédéterminée, d'autre part avec une partie de chaque fruit la plus colorée orientée vers le haut.

Elle vise également à proposer un tel procédé qui puisse être applicable à tout fruit ombiliqué, mono-ombiliqué ou bi-ombiliqué, pédonculé ou non.

Dans tout le texte, l'expression « au moins sensiblement » indique, de façon habituelle, qu'une caractéristique structurelle telle qu'une valeur, ou fonctionnelle, ne doit pas être prise comme marquant une discontinuité abrupte, qui n'aurait pas de sens physique, mais couvre non seulement cette structure ou cette fonction, mais également des variations légères de cette structure ou de cette fonction qui produisent, dans le contexte technique considéré, un effet de même nature, sinon de même degré. En outre, le terme « parallèle » est utilisé dans tout le texte comme englobant le cas de droites confondues ou de plans confondus.

L'invention concerne donc un procédé d'orientation d'un fruit ombiliqué dans lequel :
- lors d'une première phase d'orientation le fruit est supporté et entraîné en rotation propre autour d'un premier axe de rotation,
- lors d'une deuxième phase d'orientation ultérieure le fruit est supporté et entraîné en rotation propre autour d'un deuxième axe de rotation orthogonal au premier axe de rotation,
- une analyse optique d'une surface supérieure du fruit est réalisée au moins pendant au moins une partie de la première phase d'orientation à l'aide d'au moins une caméra disposée au-dessus du fruit réalisant des images de ladite surface supérieure du fruit, ces images étant transmises à une unité de traitement d'images adaptée pour analyser ces images et produire des résultats d'analyse optique dépendant de l'orientation du fruit,
- la rotation du fruit autour de chacun des deux axes de rotation est commandée en fonction au moins desdits résultats d'analyse optique du fruit, caractérisé en ce que :
- la première phase d'orientation comprend les étapes suivantes :
   ∘ une étape d'analyse optique initiale lors de laquelle :
      ▪ au moins une image, dite image initiale, du fruit est réalisée selon un axe optique de prise de vues non parallèle au premier axe de rotation,
      ▪ chaque image initiale est analysée par analyse optique, la présence d'au moins une portion d'un ombilic étant détectée dans chaque image initiale,
   ∘ puis une étape de rotation lors de laquelle le fruit est entraîné en rotation propre autour du premier axe de rotation selon une amplitude angulaire comprise entre 5° et 45°,
   ∘ puis une étape d'analyse optique ultérieure lors de laquelle :
      ▪ au moins une image, dite image ultérieure, du fruit est réalisée selon le même axe optique de prise de vues que l'image initiale,
      ▪ chaque image ultérieure est analysée par analyse optique, la présence d'au moins une portion d'un ombilic étant détectée dans chaque image ultérieure,
- l'unité de traitement exécute une étape de décision conditionnelle selon laquelle, si une première condition est satisfaite par les résultats d'analyse optique de chaque image initiale et de chaque image ultérieure, la première phase d'orientation est arrêtée et le procédé est poursuivi, ladite première condition étant satisfaite si au moins une portion d'un ombilic est détectée dans au moins une image initiale et n'est plus détectée dans chaque image ultérieure.

Il s'est avéré après de nombreux essais infructueux que la combinaison de caractéristiques d'un procédé selon l'invention permet, de façon surprenante, d'obtenir une fiabilité quasi parfaite d'orientation des fruits, et ce aussi bien avec des pommes, des poires, des tomates... En effet, la réalisation d'au moins deux images successives séparées d'une rotation du fruit autour du premier axe de rotation et l'étape de décision conditionnelle permettent en particulier d'orienter chaque ombilic du fruit dans un plan prédéterminé, dit plan de première orientation, contenant le premier axe de rotation et non parallèle -notamment au moins sensiblement perpendiculaire- à l'axe optique de prise de vues de la caméra réalisant les images initiale et ultérieure.

Un procédé selon l'invention peut faire l'objet de diverses variantes en ce qui concerne les étapes exécutées après la première phase d'orientation si ladite première condition est satisfaite (des étapes ultérieures pouvant être prévues notamment pour orienter chaque ombilic selon une direction privilégiée dudit plan de première orientation et/ou pour orienter une portion plus colorée du fruit vers le haut et/ou pour orienter le pédoncule du fruit dans un sens prédéterminé) et en ce qui concerne les étapes exécutées après l'étape de décision conditionnelle si ladite première condition n'est pas satisfaite (en vue d'optimiser l'orientation de chaque ombilic du fruit dans ledit plan perpendiculaire à l'axe optique de prise de vues). Par exemple, d'autres dispositifs d'analyse optique peuvent être prévus, pour réaliser des analyses optiques selon d'autres angles de prise de vues. Cela étant, c'est un avantage de l'invention que de permettre, dans certains modes de réalisation, l'orientation précise et finale du fruit sans adjonction d'un dispositif d'analyse optique supplémentaire, et de façon simple, fiable et efficace.

Ainsi, dans certains modes de réalisation avantageux d'un procédé d'orientation selon l'invention si ladite première condition n'est pas satisfaite, les étapes de rotation et d'analyse optique ultérieure de la première phase d'orientation sont réitérées, puis l'étape de décision conditionnelle est réitérée par l'unité de traitement en considérant l'image ultérieure réalisée avant réitération des étapes de rotation et d'analyse optique ultérieure en tant qu'image initiale.

Plus particulièrement, avantageusement et selon l'invention, selon l'étape de décision conditionnelle :
- si au moins une portion d'un ombilic est détectée dans au moins une image initiale et dans au moins une image ultérieure, les étapes de rotation et d'analyse optique ultérieure de la première phase d'orientation sont réitérées,
- si au moins une portion d'un ombilic n'est détectée ni dans chaque image initiale ni dans chaque image ultérieure, les étapes de rotation et d'analyse optique ultérieure de la première phase d'orientation sont réitérées, tant que l'amplitude angulaire totale de la rotation du fruit résultant des différentes étapes de rotation effectuées lors de la première phase d'orientation est inférieure à une amplitude angulaire, dite amplitude de rotation maximum, prédéterminée comprise entre 180° et 360° -notamment de l'ordre de 270 °-,
- si au moins une portion d'un ombilic n'est détectée ni dans chaque image initiale ni dans chaque image ultérieure, les étapes de rotation et d'analyse optique ultérieure de la première phase d'orientation sont réitérées, et si l'amplitude angulaire totale de la rotation du fruit résultant des différentes étapes de rotation précédemment effectuées lors de la première phase d'orientation est supérieure ou égale à ladite amplitude de rotation maximum, la première phase d'orientation est arrêtée et le procédé est poursuivi,
- si au moins une portion d'un ombilic n'est pas détectée dans chaque image initiale mais est détectée dans au moins une image ultérieure, les étapes de rotation et d'analyse optique ultérieure de la première phase d'orientation sont réitérées, puis l'étape de décision conditionnelle est réitérée par l'unité de traitement en considérant l'image ultérieure réalisée avant réitération des étapes de rotation et d'analyse optique ultérieure en tant qu'image initiale.

Un tel procédé d'orientation selon l'invention permet de garantir une convergence rapide d'orientation de chaque ombilic du fruit dans un plan de première orientation au moins sensiblement perpendiculaire à l'axe optique de prise de vues de chaque image initiale et de chaque image ultérieure réalisées au cours de ladite première phase d'orientation, notamment dans un plan de première orientation au moins sensiblement -notamment strictement- horizontal.

Ledit axe optique de prise de vues ne doit pas être parallèle au premier axe de rotation, de façon que chaque image ultérieure soit une image d'une portion de surface supérieure du fruit distincte de celle vue par chaque image initiale. Ledit axe de prise de vues peut être incliné par rapport au premier axe de rotation. Néanmoins, de préférence, ledit axe optique de prise de vues est au moins sensiblement -notamment strictement- orthogonal, de préférence au moins sensiblement -notamment strictement- perpendiculaire, au premier axe de rotation. Il est à noter cependant que rien n'empêche, dans certaines variantes de réalisation, de prévoir plusieurs caméras pour former plusieurs images initiales (ou plusieurs images ultérieures), dans des spectres de longueur d'onde différents ou dans les mêmes spectres de longueur d'onde, ces caméras étant orientées selon des axes de prise de vues au moins sensiblement parallèles mais distants l'un de l'autre, par exemple passant de part et d'autre du premier axe de rotation. Rien n'empêche également de réaliser une image initiale (ou une image ultérieure) par combinaison de plusieurs images réalisées par plusieurs caméras, par exemple en formant par calcul une moyenne de plusieurs images d'origine réalisées selon des axes optique de prise de vues au moins sensiblement parallèles (ledit axe optique de prise de vues de l'image initiale ou de l'image ultérieure étant alors un axe optique virtuel parallèle aux axes optiques de prise de vues des images d'origine, et situé entre ces derniers, à même distance de ces derniers, notamment en position de médiatrice).

Par ailleurs, le premier axe de rotation étant un axe de rotation propre du fruit est un axe qui passe par le fruit, de préférence au moins sensiblement par un centre géométrique ou un centre de gravité du fruit. Il est à noter cependant que ce premier axe de rotation peut être défini de façon uniquement approximative par rapport au fruit, selon la forme et les dimensions de la surface extérieure du fruit, lorsque ce dernier est entraîné en rotation propre par des rouleaux supportant le fruit. De même, le deuxième axe de rotation étant un axe de rotation propre du fruit est un axe qui passe par le fruit, de préférence au moins sensiblement par un centre géométrique ou un centre de gravité du fruit. En conséquence, ces deux axes de rotation sont de préférence au moins sensiblement sécants en un point passant par le fruit, de préférence au moins sensiblement en un centre géométrique ou un centre de gravité du fruit. Ainsi, les deux axes de rotation propre du fruit ne sont pas seulement orthogonaux l'un à l'autre, mais également au moins sensiblement perpendiculaires l'un à l'autre.

Dans certains modes de réalisation, avantageusement et selon l'invention, le deuxième axe de rotation est perpendiculaire à un plan, dit plan de première orientation, contenant le premier axe de rotation et non parallèle à l'axe optique de prise de vues. Dans les modes de réalisation préférentiels dans lesquels l'axe optique de prise de vues est au moins sensiblement -notamment strictement-orthogonal au premier axe de rotation, avantageusement et selon l'invention le deuxième axe de rotation est au moins sensiblement -notamment strictement-parallèle audit axe optique de prise de vues. Dans les modes de réalisation préférentiels dans lesquels l'axe optique de prise de vues est au moins sensiblement -notamment strictement- perpendiculaire au premier axe de rotation, avantageusement et selon l'invention le deuxième axe de rotation est au moins sensiblement -notamment strictement- confondu avec ledit axe optique de prise de vues.

Par ailleurs, dans certains modes de réalisation préférentiels, le premier axe de rotation est contenu dans un plan horizontal et le deuxième axe de rotation est vertical. En outre, de préférence, ledit axe optique de prise de vues est alors également vertical.

Par ailleurs, l'étape de rotation de la première phase d'orientation d'un procédé selon l'invention peut faire l'objet de diverses variantes, notamment pour minimiser la durée de cette étape de rotation et de l'ensemble du procédé selon l'invention, et ce en particulier en fonction des caractéristiques géométriques et dimensionnelles des fruits.

Ainsi, en particulier, lors de cette étape de rotation le fruit est entraîné en rotation selon une amplitude angulaire comprise entre 10° et 20°, en particulier de l'ordre de 15° pour des pommes, cette valeur d'amplitude angulaire dépendant en particulier de la technique d'analyse optique utilisée pour détecter un ombilic dans les images et des dimensions relatives moyennes de la dépression ombilicale de chaque ombilic du fruit. La valeur optimale peut être déterminée expérimentalement. Elle doit être en particulier suffisamment faible pour garantir que le fruit soit effectivement déplacé (sans glissement), et procurer une précision suffisante de déplacement de telle sorte que l'ombilic soit correctement orienté après la rotation si seulement une portion de cet ombilic est détectée dans l'image initiale. Elle est suffisamment grande pour optimiser la durée de cette étape et de l'ensemble du procédé.

De même, dans certains modes de réalisation avantageux, ladite étape d'analyse optique initiale comprend une détection d'un centre du fruit dans au moins une image initiale, et si au moins une portion d'un ombilic est détectée dans au moins une image initiale, lors de l'étape de rotation subséquente le fruit est entraîné en rotation dans un sens déterminé par les positions respectives du centre du fruit et de l'ombilic détectés, et choisi pour minimiser l'amplitude angulaire de déplacement de l'ombilic vers un plan, dit plan de première orientation, contenant le premier axe de rotation et non parallèle à l'axe optique de prise de vues. Dans les modes de réalisation préférentiels mentionnés ci-dessus, ledit plan de première orientation est un plan au moins sensiblement -notamment strictement- perpendiculaire audit axe optique de prise de vues et contenant le premier axe de rotation.

Toute technique d'imagerie appropriée peut être utilisée pour la détection d'un ombilic dans un fruit lors d'une étape d'analyse optique d'un procédé selon l'invention. Cela étant, il a été constaté que des résultats étonnamment fiables ont été obtenus par analyse des niveaux de gris en imagerie infrarouge. Ainsi, avantageusement et selon l'invention, lors de chaque étape d'analyse optique, au moins une image, dite image infrarouge, est réalisée à l'aide d'au moins caméra infrarouge, et la présence d'au moins une portion d'un ombilic dans chaque image infrarouge sous forme d'une tâche présentant un niveau de gris supérieur à un niveau de gris prédéterminé et de dimension inférieure à celle du fruit mais supérieure à une dimension prédéterminée. Cette tâche peut être détectée dans l'image infrarouge par toute technique appropriée d'analyse d'image, notamment à l'aide d'un algorithme comprenant une convolution et un noyau de convolution. Tel est en particulier le cas pour chaque étape d'analyse optique de la première phase d'orientation d'un procédé selon l'invention. En effet, il s'avère qu'un ombilic d'un fruit est représenté en imagerie infrarouge par une tâche plus sombre.

Avantageusement et selon l'invention, le fruit est éclairé par une source de lumière infrarouge dont la longueur d'onde est supérieure à 500 nm et inférieure à 1100 nm, par exemple de l'ordre de 740 nm, et on utilise au moins une caméra infrarouge permettant de détecter les longueurs d'onde autour de celle de la source de lumière infrarouge, par exemple une caméra infrarouge sensible entre 350nm et 1100nm avec un filtre passe-haut présentant une longueur d'onde de coupure légèrement inférieure à celle de la source de lumière infrarouge, par exemple de l'ordre de 700 nm.

Comme indiqué ci-dessus, les étapes exécutées après la première phase d'orientation d'un procédé selon l'invention peuvent faire l'objet de plusieurs variantes. Dans certains modes de réalisation préférentiels, avantageusement et selon l'invention à la fin de la première phase d'orientation l'unité de traitement :
- identifie la dernière image réalisée dans laquelle au moins une portion d'un ombilic est détectée par analyse optique, détermine la position d'un centre du fruit dans cette dernière image, et calcule la valeur d'un angle, dit azimut, formé entre le premier axe de rotation et un axe, dit axe ombilical, passant par l'ombilic et le centre du fruit détectés,
- puis commande une rotation du fruit autour du deuxième axe de rotation lors de la deuxième phase d'orientation selon une amplitude angulaire déterminée par la valeur calculée d'azimut de façon à orienter l'axe ombilical selon une orientation prédéterminée par rapport au premier axe de rotation, en particulier soit au moins sensiblement parallèlement au premier axe de rotation -notamment strictement parallèlement au premier axe de rotation-, soit au moins sensiblement orthogonalement au premier axe de rotation -notamment strictement orthogonalement au premier axe de rotation-.

En outre, un procédé d'orientation selon l'invention comprend également avantageusement une étape d'optimisation colorimétrique permettant de placer les portions les plus colorées des fruits dans une même position prédéterminée. Ainsi, avantageusement un procédé selon l'invention comprend, après la deuxième phase d'orientation, une phase ultérieure d'orientation lors de laquelle :
- le fruit est supporté et entraîné en rotation sur une amplitude angulaire de rotation d'au moins 360° autour du premier axe de rotation,
- une analyse optique d'une surface supérieure du fruit est réalisée pour détecter une portion de cette surface supérieure, dite portion la plus colorée, présentant une coloration maximum,
- la rotation du fruit est interrompue de façon à placer ladite portion la plus colorée sur le dessus.

Dans certaines variantes d'un procédé selon l'invention rien n'empêche de prévoir des étapes différentes des étapes susmentionnées et/ou des étapes intermédiaires entre les étapes susmentionnées. Par exemple, il peut être prévu au moins une étape d'analyse morphologique dimensionnelle avant rotation selon le deuxième axe de rotation et/ou au moins une étape de recherche d'un défaut morphologique avant ou après l'étape d'optimisation colorimétrique.

L'invention s'applique à l'orientation de fruits comprenant un seul ombilic, ou plusieurs ombilics (fruits dits bi-ombiliqués), à savoir une cuvette oculaire et une cavité pédonculaire, avec ou sans pédoncule.

L'invention concerne cependant plus particulièrement un procédé d'orientation d'un fruit pédonculé caractérisé en ce qu'il comprend, après la deuxième phase d'orientation, une étape d'analyse optique morphologique adaptée pour permettre une détection de la position d'un pédoncule du fruit. Une telle étape d'analyse optique morphologique permet de distinguer la position du pédoncule par rapport à celle de la cuvette oculaire. Elle peut être par exemple exécutée dans le cas de pommes à partir des images réalisées et par détection de la position du plus grande diamètre du fruit par rapport à un diamètre du fruit passant par le centre du fruit perpendiculairement à l'axe ombilical du fruit, le pédoncule étant situé du côté du plus grand diamètre du fruit.

La position du pédoncule du fruit ainsi détectée peut être simplement enregistrée et prise en compte ultérieurement, par exemple par un robot de manutention pour orienter correctement le fruit dans un emballage alvéolé. Cela étant, dans certains modes de réalisation, le procédé selon l'invention comprend en outre après l'étape d'analyse optique morphologique, une étape ultérieure de rotation lors de laquelle le fruit est entraîné en rotation autour du deuxième axe de rotation selon une amplitude angulaire déterminée pour placer le pédoncule dans une position angulaire prédéterminée par rapport au premier axe de rotation.

L'invention s'étend également à un dispositif d'orientation adapté pour la mise en œuvre d'un procédé d'orientation selon l'invention. L'invention concerne donc également un dispositif d'orientation d'un fruit ombiliqué comprenant :
- un premier support de fruit adapté pour supporter un fruit et pour l'entraîner en rotation propre autour d'un premier axe de rotation,
- un deuxième support de fruit adapté pour supporter un fruit et pour l'entraîner en rotation propre autour d'un deuxième axe de rotation orthogonal au premier axe de rotation,
- un dispositif d'analyse optique d'une surface supérieure du fruit comprenant au moins une caméra disposée au-dessus du fruit pour pouvoir réaliser des images de ladite surface supérieure du fruit,
- une unité de traitement programmable adapté pour :
   ∘ analyser les images et produire des résultats d'analyse optique dépendant de l'orientation du fruit,
   ∘ commander la rotation du fruit autour de chacun des deux axes de rotation en fonction aux moins desdits résultats d'analyse optique du fruit,
   caractérisé en ce que ladite unité de traitement programmable est programmée pour mettre en œuvre un procédé d'orientation selon l'invention.

L'invention s'étend également en particulier à un procédé d'emballage de fruits ombiliqués dans des emballages alvéolés dans lequel chaque fruit est placé dans une alvéole d'emballage selon une orientation prédéterminée caractérisé en ce qu'il comprend un procédé d'orientation de chaque fruit selon l'invention.

L'invention s'étend également à un dispositif d'emballage pour la mise en œuvre d'un procédé d'emballage selon l'invention. Elle concerne donc également un dispositif d'emballage de fruits ombiliqués comprenant des dispositifs d'orientation des fruits et au moins un robot de manutention des fruits adapté pour placer chaque fruit dans une alvéole d'un emballage alvéolé selon une orientation prédéterminée caractérisé en ce qu'il comprend au moins un dispositif d'orientation selon l'invention.

L'unité de traitement programmable décrite dans le présent texte peut être formée de tout ou partie d'un système informatique programmable pouvant être mis en œuvre par un programme d'ordinateur ou une pluralité de programmes d'ordinateur, qui peuvent exister sous diverses formes, à la fois active et inactive, dans un système informatique unique ou une pluralité de systèmes informatiques. Par exemple, ils peuvent consister en des programmes logiciels formés d'instructions de programme en code source, code objet, code exécutable ou autre format pour exécuter au moins une partie des étapes d'un procédé selon l'invention. Ils peuvent se trouver sous la forme d'un flux de données téléchargeable ou d'un support lisible par ordinateur, qui inclut des dispositifs d'enregistrement et des signaux, sous forme comprimée ou non comprimée.

L'invention s'étend ainsi à un programme d'ordinateur comprenant des instructions de code de programme informatique -notamment des instructions de code de programme informatique constituant un flux de données téléchargeable et/ou des instructions enregistrées sur un support utilisable dans une unité de traitement programmable- caractérisé en ce qu'il comprend des moyens de programmation lisibles par une unité de traitement programmable, et adaptés pour, une fois exécutés par ladite unité de traitement programmable, exécuter un procédé d'orientation selon l'invention et/ou un procédé d'emballage selon l'invention avec ladite unité de traitement programmable et avec un dispositif d'orientation de chaque fruit adapté pour supporter et entraîner chaque fruit en rotation propre autour dudit premier axe de rotation et autour dudit deuxième axe de rotation -notamment avec un dispositif d'orientation selon l'invention-.

L'invention s'étend également à un programme d'ordinateur comprenant des instructions de code de programme informatique -notamment des instructions de code de programme informatique constituant un flux de données téléchargeable et/ou des instructions enregistrées sur un support utilisable dans un système programmable informatique et/ou robotique-, caractérisé en ce qu'il comprend des moyens de programmation lisibles par un système programmable informatique et/ou robotique, et adaptés pour, une fois exécutés par une unité de traitement programmable dudit système programmable informatique et/ou robotique (notamment chargés en mémoire de cette unité de traitement programmable), exécuter un procédé d'orientation selon l'invention et/ou un procédé d'emballage selon l'invention avec ladite unité de traitement programmable et avec un dispositif d'orientation de chaque fruit adapté pour supporter et entraîner chaque fruit en rotation propre autour dudit premier axe de rotation et autour dudit deuxième axe de rotation.

L'invention s'étend aussi à un produit programme d'ordinateur comprenant des instructions de code de programme informatique caractérisé en ce qu'il comprend des moyens de programmation lisibles par une unité de traitement programmable d'un système informatique et/ou robotique, lesdits moyens de programmation étant adaptés pour, une fois exécutés par ladite unité de traitement programmable, exécuter un procédé d'orientation selon l'invention et/ou un procédé d'emballage selon l'invention avec ladite unité de traitement programmable et avec un dispositif d'orientation de chaque fruit adapté pour supporter et entraîner chaque fruit en rotation propre autour dudit premier axe de rotation et autour dudit deuxième axe de rotation -notamment avec un dispositif d'orientation selon l'invention-.

L'invention s'étend encore à support utilisable dans un système programmable informatique et/ou robotique, ce support comprenant des instructions de code d'un programme informatique enregistrées sur ce support et utilisables dans une unité de traitement programmable d'un tel système programmable informatique et/ou robotique, caractérisé en ce qu'il comprend, enregistrés sur ce support, des moyens de programmation lisibles par une unité de traitement programmable d'un système informatique et/ou robotique, lesdits moyens de programmation étant adaptés pour, une fois exécutés par ladite unité de traitement programmable, exécuter un procédé d'orientation selon l'invention et/ou un procédé d'emballage selon l'invention avec ladite unité de traitement programmable et avec un dispositif d'orientation de chaque fruit adapté pour supporter et entraîner chaque fruit en rotation propre autour dudit premier axe de rotation et autour dudit deuxième axe de rotation -notamment avec un dispositif d'orientation selon l'invention-.

Telle qu'elle est utilisée dans le présent texte, l'expression « support utilisable dans un système programmable informatique et/ou robotique » peut se référer à tout dispositif qui peut contenir, mémoriser, communiquer, propager ou transporter un programme pour son utilisation par ou en connexion avec un tel système programmable informatique et/ou robotique, un terminal, un appareil ou un dispositif d'exécution d'instructions de code de programme. Un tel support utilisable dans un système programmable informatique et/ou robotique peut être, à titre d'exemple non limitatif, un terminal, un dispositif, un appareil, un système ou un milieu de propagation électronique, magnétique, optique, électromagnétique, infrarouge ou semi-conducteur. Certains exemples spécifiques non exhaustifs d'un tel support peuvent être les suivants : un terminal informatique, une connexion électrique ayant un ou plusieurs conducteurs, une mémoire de masse (disque dur, clé USB...), une disquette, une mémoire à accès aléatoire (RAM), une mémoire à lecture seule (ROM), une mémoire à lecture seule effaçable par programmation (EPROM ou mémoire flash), une fibre optique, une mémoire à lecture optique (disque compact à lecture seule ou réinscriptible). L'invention s'étend également à un flux de données téléchargeable représentatif d'un programme d'ordinateur selon l'invention.

L'invention concerne également un procédé d'orientation, un dispositif d'orientation, un procédé d'emballage, un dispositif d'emballage, un programme d'ordinateur, un produit programme d'ordinateur, un support utilisable dans un système programmable informatique et/ou robotique caractérisés en combinaison par tout ou partie des caractéristiques mentionnées ci-dessus ou ci-après.

D'autres buts, caractéristiques et avantages de l'invention apparaîtront à la lecture de la description suivante donnée à titre non limitatif et qui se réfère aux figures annexées dans lesquelles :
- la figure 1 est un schéma illustrant un dispositif d'orientation selon un mode de réalisation de l'invention,
- la figure 2a est une vue schématique en perspective illustrant un support de fruit d'un dispositif d'orientation selon un mode de réalisation de l'invention, la tige élévatrice d'entraînement en rotation selon le deuxième axe de rotation étant déployée, le fruit étant porté par cette tige élévatrice,
- la figure 2b est une vue schématique en perspective similaire à la figure 2a, mais vue du côté opposé, la tige élévatrice d'entraînement en rotation selon le deuxième axe de rotation étant rétractée, le fruit étant porté par les rouleaux du support,
- la figure 3 est une vue schématique de dessus illustrant les caractéristiques géométriques d'un fruit telles que vues par un dispositif d'analyse optique d'un dispositif d'orientation selon un mode de réalisation de l'invention,
- les figures 4a et 4b sont des vues schématiques en élévation et, respectivement de dessus, d'un support de fruit d'un dispositif d'orientation illustrant un premier exemple d'étape de rotation d'une première phase d'orientation d'un procédé d'orientation selon un mode de réalisation de l'invention,
- les figures 5a et 5b sont des vues schématiques en élévation et, respectivement de dessus, d'un support de fruit d'un dispositif d'orientation illustrant un deuxième exemple d'étape de rotation d'une première phase d'orientation d'un procédé d'orientation selon un mode de réalisation de l'invention,
- les figures 6a et 6b sont des vues schématiques en élévation et, respectivement de dessus, d'un support de fruit d'un dispositif d'orientation illustrant un premier exemple d'orientation d'un fruit à l'issue d'une première phase d'orientation d'un procédé d'orientation selon un mode de réalisation de l'invention,
- les figures 7a et 7b sont des vues schématiques en élévation et, respectivement de dessus, d'un support de fruit d'un dispositif d'orientation illustrant un deuxième exemple d'orientation d'un fruit à l'issue d'une première phase d'orientation d'un procédé d'orientation selon un mode de réalisation de l'invention,
- la figure 8 est une vue schématique en élévation d'un support de fruit d'un dispositif d'orientation au cours d'une deuxième phase d'orientation d'un procédé d'orientation selon un mode de réalisation de l'invention,
- la figure 9 est une vue schématique de dessus d'un support de fruit d'un dispositif d'orientation à la fin d'une deuxième phase d'orientation d'un procédé d'orientation selon un mode de réalisation de l'invention,
- la figure 10 est une vue schématique en élévation d'un support de fruit d'un dispositif d'orientation au cours d'une troisième phase d'orientation d'un procédé d'orientation selon un mode de réalisation de l'invention,
- la figure 11 est une vue schématique de dessus de la figure 10,
- la figure 12 est une vue schématique de dessus d'un support de fruit d'un dispositif d'orientation à la fin de la troisième phase d'orientation d'un procédé d'orientation selon un mode de réalisation de l'invention,
- la figure 13 est une vue schématique en élévation d'un support de fruit d'un dispositif d'orientation au cours d'une quatrième phase d'orientation d'un procédé d'orientation selon un mode de réalisation de l'invention,
- les figures 14a et 14b sont des vues schématiques de dessus d'un support de fruit d'un dispositif d'orientation au début et, respectivement à la fin d'un premier exemple de la quatrième phase d'orientation d'un procédé d'orientation selon un mode de réalisation de l'invention,
- les figures 15a et 15b sont des vues schématiques de dessus d'un support de fruit d'un dispositif d'orientation au début et, respectivement à la fin d'un deuxième exemple de la quatrième phase d'orientation d'un procédé d'orientation selon un mode de réalisation de l'invention,
- la figure 16 est un exemple d'image d'un fruit permettant une analyse morphologique de ce dernier,
- la figure 17 est un organigramme d'un procédé d'emballage selon un mode de réalisation de l'invention,
- la figure 18 est un organigramme de la première phase d'orientation d'un procédé d'orientation selon un mode de réalisation de l'invention,
- la figure 19 est une vue en élévation représentant un dispositif d'emballage selon l'invention,
- la figure 20 est une vue de profil du dispositif de la figure 19.

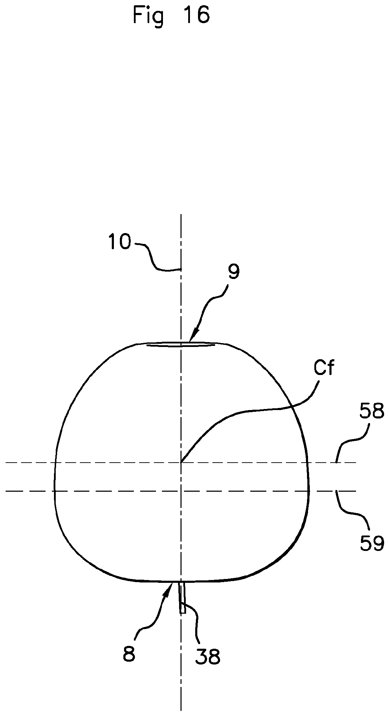

Un fruit ombiliqué tel qu'une pomme présente au moins un ombilic déterminant un axe, dit axe 10 ombilical (figure 16), par rapport auquel le fruit est globalement au moins sensiblement symétrique de révolution. Un fruit ombiliqué peut présenter un seul ombilic correspondant en général à une cavité pédonculaire (pêches, abricots...) ; ou au contraire, comme dans le cas des pommes, deux ombilics 8, 9 opposés l'un 8 correspondant à une cavité pédonculaire, l'autre 9 correspondant à une cuvette oculaire, l'axe 10 ombilical passant par les deux ombilics opposés 8, 9. Un procédé selon l'invention d'orientation d'un fruit ombiliqué présente les principales étapes suivantes.

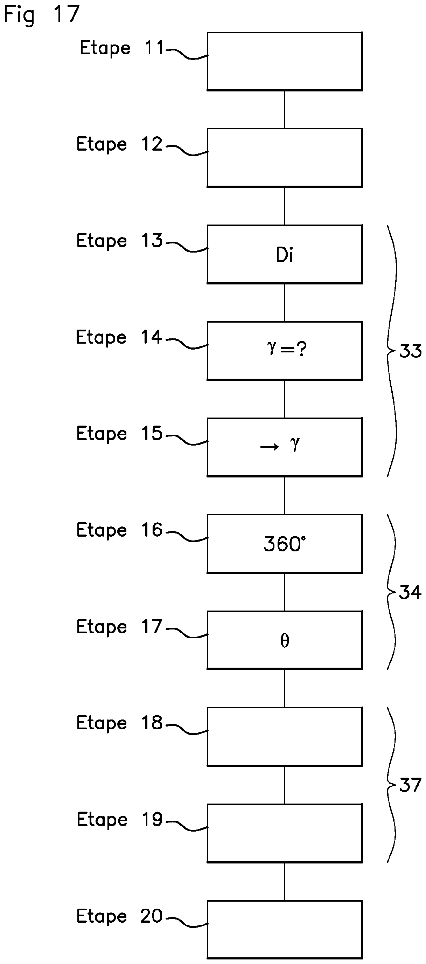

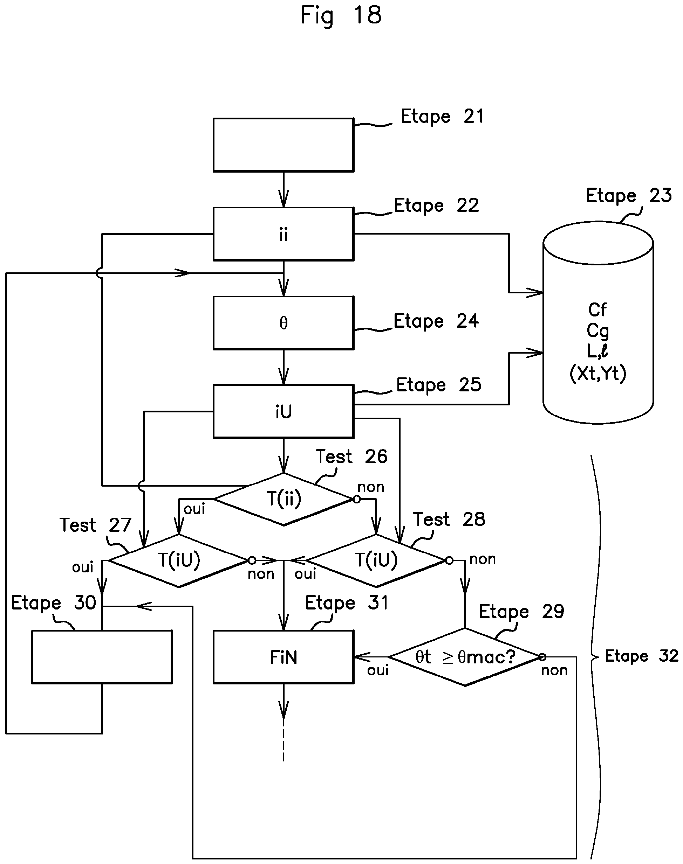

Sur les figures 17 et 18, les rectangles et les losanges identifient les étapes et les tests, qui sont identifiés par les références numériques mentionnées ci-après. Les tests sont représentés conformément à la norme ISO 5807.

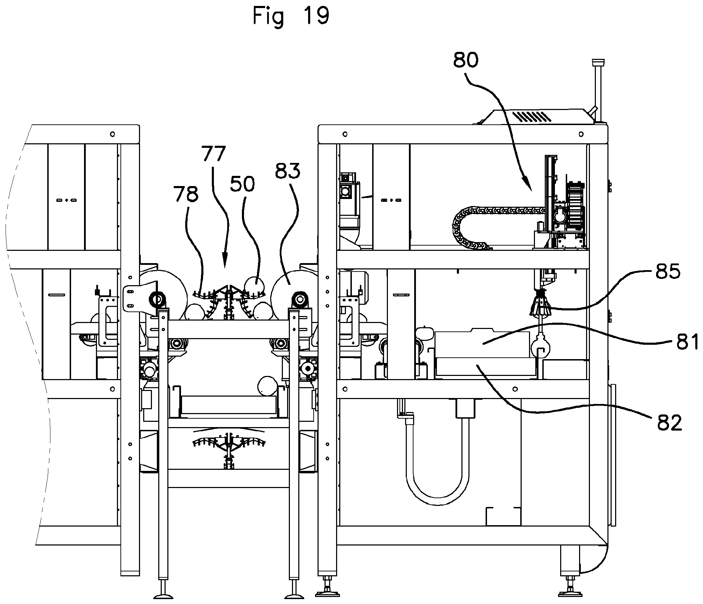

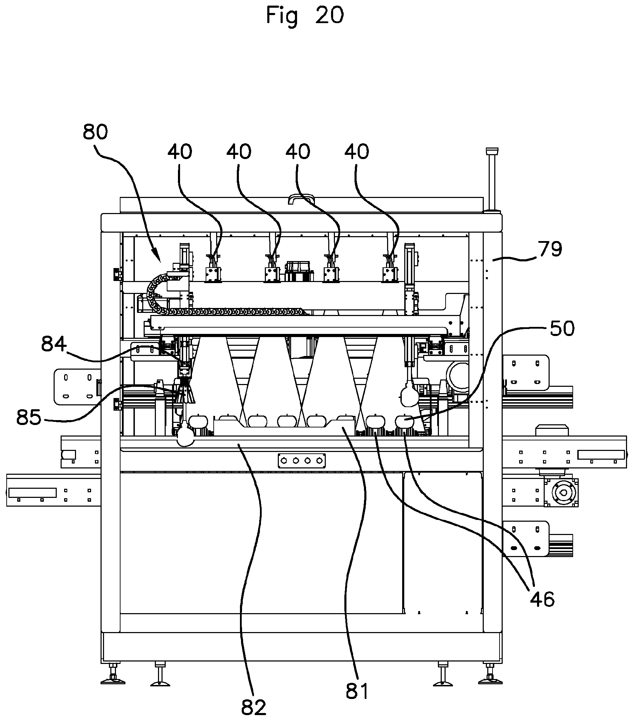

Dans une première étape 11 de chargement, un fruit ombiliqué est chargé sur un dispositif d'orientation d'un fruit ombiliqué selon l'invention. Ce fruit ombiliqué peut être fourni à la sortie d'une unité de calibrage telle que par exemple décrite par US5626238 ou EP0670276. De la sorte, le calibre moyen, c'est-à-dire le diamètre moyen, du fruit ombiliqué est déterminé. Le chargement d'un fruit sur un dispositif d'orientation peut être effectué par tous moyens appropriés transférant des fruits sur un dispositif d'orientation d'un banc formé d'une pluralité de dispositifs d'orientation qui se jouxtent en sortie de l'unité de calibrage (figures 19 et 20).

En variante, le dispositif d'orientation peut être intégré à un convoyeur comprenant une pluralité de dispositifs d'orientation entraînés en boucle, ce convoyeur étant entraîné en synchronisme avec un autre convoyeur de transport des fruits (par exemple un convoyeur d'une unité de calibrage des fruits) pour réaliser en ligne l'orientation des fruits (comme par exemple décrit par EP 1183197). Dans ce cas l'étape 11 de chargement est une étape de prise en charge d'un fruit par un dispositif d'orientation.

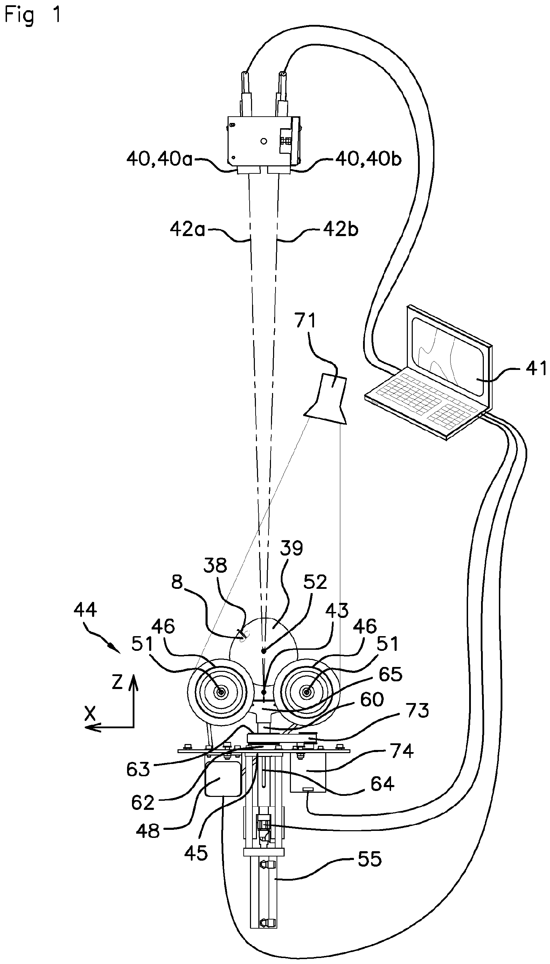

Chaque dispositif d'orientation selon l'invention comprend en particulier un dispositif d'analyse optique comprenant au moins une caméra 40 agencée pour pouvoir réaliser des images d'un fruit ombiliqué chargé sur le dispositif d'orientation, et une unité 41 informatique de traitement recevant les signaux délivrés par chaque caméra 40 et adaptée pour pouvoir analyser les images formées par cette dernière, et en particulier pour détecter la présence ou non d'un fruit ombiliqué sur le dispositif d'orientation. Cette unité 41 informatique de traitement est avantageusement une unité informatique de traitement de données numériques, chaque caméra 40 fournissant des données numériques représentatives des images du fruit. Dans l'exemple représenté figure 1, le dispositif d'orientation comprend deux caméras 40a, 40b, par exemple l'une 40a réalisant des images dans le domaine visible, et l'autre 40b réalisant des images dans le domaine des infrarouges.

Les deux caméras 40a, 40b se jouxtent de telle sorte que leurs axes 42a, 42b optique de prise de vues respectifs sont très proches l'un de l'autre et convergent en un point 43 d'un support 44 du dispositif d'orientation permettant de supporter un fruit et de l'entraîner en rotation propre.

Par exemple, le fruit est éclairé par une source 71 de lumière infrarouge orientée vers le support 44 vers le fruit et dont la longueur d'onde est de l'ordre de 740 nm, et la caméra 40b infrarouge est une caméra sensible aux longueurs d'onde entre 350nm et 1100nm associés à un filtre passe-haut présentant une longueur d'onde de coupure de l'ordre de 695 nm. La caméra 40a sensible dans les longueurs d'onde du domaine visible est munie avantageusement d'un filtre passe bande dont la bande de longueur d'onde est par exemple comprise entre 390 nm et 690 nm.

Si la présence d'un fruit sur le support 44 d'un dispositif d'orientation est détectée par le dispositif d'analyse optique, le support 44 est commandé par l'unité 41 informatique de traitement de façon à exécuter des étapes d'orientation du fruit comme décrit plus en détail ci-après.

Le support 44 d'un dispositif d'orientation comprend une platine 45 horizontale portant deux rouleaux 46 montés rotatifs sur des arbres 47 supportés et guidés en rotation par rapport à la platine 45 selon des axes 51 de rotation horizontaux parallèles l'un à l'autre. Les deux rouleaux 46 sont entraînés en rotation dans le même sens de rotation par un moteur électrique 48 via une courroie 49. Ils sont espacés l'un de l'autre d'une distance adaptée pour permettre de supporter entre eux un unique fruit 50. La rotation des rouleaux 46 entraîne donc la rotation propre du fruit 50 roulant sur lui-même sur les rouleaux 46 autour d'un premier axe 52 de rotation parallèle aux axes 51 de rotation des rouleaux 46.

Les axes 51 de rotation des rouleaux 46 définissent un plan horizontal dans lequel peuvent être définis un axe X perpendiculaire aux axes 51 de rotation des rouleaux 46, et un axe Y parallèle aux axes 51 de rotation des rouleaux 46. La direction verticale perpendiculaire à ce plan horizontal et aux axes X et Y définit un axe Z vertical, les axes X, Y, Z définissant un repère orthonormé représenté sur les figures.

Le support 44 porte également une tige 60 élévatrice interposée à mi-distance entre les deux axes 51 de rotation des rouleaux 46 et qui s'étend verticalement et vers le haut entre les deux rouleaux 46 orthogonalement aux axes 51 de rotation de ces rouleaux 46. La tige 60 élévatrice est guidée et entraînée en rotation autour de son axe 61 vertical par rapport à la platine 45 du support 44 qu'elle traverse. Pour ce faire, la platine 45 porte un roulement 62 guidant en rotation une roue 63 présentant un alésage interne traversant à clavette(s) ou rainure(s) traversé par la tige 60 élévatrice. La tige 60 élévatrice présente au moins une fente 64 longitudinale adaptée pour pouvoir glisser le long d'au moins une clavette ou rainure de l'alésage interne de la roue 63, de sorte que la tige 60 élévatrice peut se déplacer en translation selon son axe 61 par rapport à la roue 63, tout en étant toujours solidaire en rotation de cette roue 63 autour de son axe 61.

La roue 63 est entraînée en rotation autour de l'axe 61 de la tige 60 élévatrice dans un sens ou dans l'autre par rapport à la platine 45 par une courroie 73 accouplée à un moteur 74 électrique porté par la platine 45.

La tige 60 élévatrice est creuse et présente donc un alésage axial traversant et porte à son extrémité supérieure une ventouse 65. Son extrémité inférieure 66 est sertie dans un coulisseau 67 de façon à pouvoir être entraînée en translation par ce coulisseau 67 et à pouvoir être entraînée en rotation autour de son axe 61 par rapport à ce coulisseau 67. Le coulisseau 67 porte un raccord pneumatique tournant 68 connecté à l'extrémité libre inférieure 66 de la tige 60 élévatrice de façon à relier cette extrémité libre inférieure 66 à une source d'air d'aspiration (non représentée), tout en autorisant la rotation de la tige 60 élévatrice autour de son axe 61 alors que le raccord 68 est fixe par rapport au coulisseau 67.

Le coulisseau 67 est guidé sous la platine 45 par quatre coulisses 69 fixées sous la platine 45 s'étendant verticalement vers le bas jusqu'à une plaque 70 de support sur laquelle est fixé un corps 55 d'un vérin 53 dont la tige 54 d'actionnement traverse verticalement la plaque 70 de support pour être connectée au coulisseau 67.

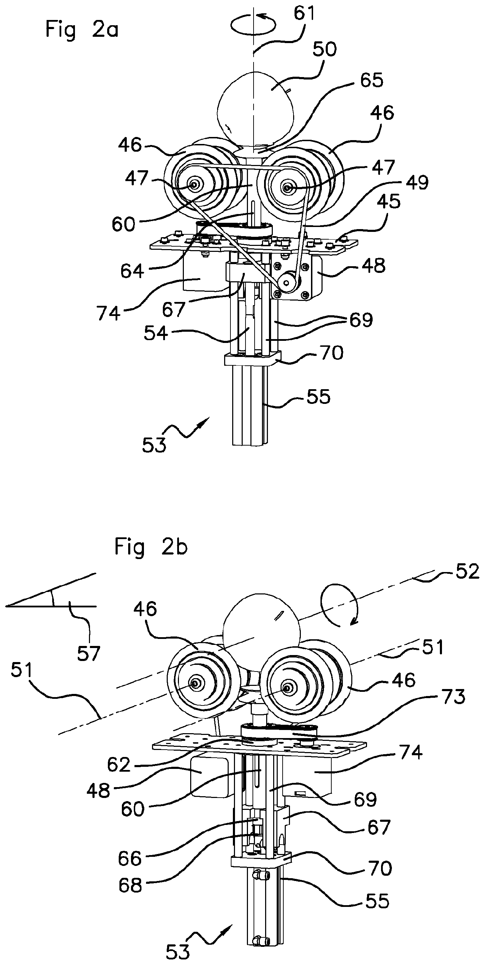

Lorsque la tige 54 d'actionnement du vérin 53 est rétractée dans le corps 55, le coulisseau 67 est en position basse contre la plaque 70 de support, la tige 60 élévatrice est rétractée vers le bas et la ventouse 65 s'étend entre les rouleaux 46 à distance d'un fruit supporté entre ces rouleaux 46 (figure 2b). La ventouse 65, qui n'est pas alimentée en air d'aspiration n'est pas en contact avec le fruit et ne coopère pas avec ce dernier. Lorsque la tige 54 d'actionnement du vérin 53 est déployée, le coulisseau 67 est en position haute immédiatement sous la platine 45, la tige 60 élévatrice est déployée vers le haut et la ventouse 65 s'étend vers le haut au-dessus des rouleaux 46 de façon à soulever et porter un fruit précédemment supporté entre les rouleaux 46. La ventouse 65 étant alimentée en air d'aspiration porte le fruit et, lorsque le moteur 74 électrique est activé et que la tige 60 élévatrice est entraînée en rotation autour de son axe vertical 61, le fruit porté par la ventouse 65 et solidaire de cette dernière est également entraîné en rotation propre autour de cet axe vertical 61. L'axe vertical 61 de la tige 60 élévatrice définit un deuxième axe 61 de rotation propre du fruit orthogonal au premier axe 52 de rotation propre du fruit. Ce deuxième axe 61 de rotation propre étant situé à mi-distance entre les axes 51 de rotation des rouleaux 46 est au moins sensiblement perpendiculaire au premier axe 52 de rotation propre du fruit. Il est à noter cependant que, dans ce mode de réalisation du dispositif d'orientation selon l'invention, si le deuxième axe 61 de rotation propre présente par construction une position et une orientation fixes par rapport aux rouleaux 46, tel n'est pas le cas du premier axe 52 de rotation propre dont la position et l'orientation sont définies uniquement de façon approximative par rapport aux rouleaux 46, compte tenu de la forme et des dimensions de la surface extérieure du fruit 50 qui roule sur les rouleaux 46. Ainsi, le deuxième axe 61 de rotation propre peut ne pas être strictement sécant avec le premier axe 52 de rotation propre, selon la forme et les dimensions particulières du fruit. Dans le mode de réalisation représenté, le premier axe 52 de rotation propre est contenu dans un plan horizontal et le deuxième axe 61 de rotation est vertical. La tige 60 élévatrice est commandée dans ses translations verticales parallèlement à l'axe Z vertical par le vérin 53.

Les axes 42a, 42b optique de prise de vues des caméras 40a, 40b sont au moins sensiblement parallèles à l'axe Z vertical, et donc au deuxième axe 61 de rotation propre du fruit, de sorte que les caméras permettent de réaliser des prises de vues d'une surface supérieure 39 du fruit 50 supporté par les rouleaux 46 ou par la tige 60 élévatrice, de sorte que l'unité 41 informatique de traitement réalise une analyse optique de cette surface 39 supérieure du fruit. Initialement, lors de l'étape 11 de chargement, la tige 60 élévatrice est en position rétractée, de sorte que lorsqu'un fruit est chargé sur le support 44, ce fruit est supporté par les rouleaux 46.

Dès qu'un fruit est détecté lors d'une étape 21 de détection du fruit sur le support 44 par l'une ou l'autre des caméras 40a, 40b, une première phase 12 d'orientation du fruit est réalisée sur commande de l'unité 41 informatique de traitement de façon à placer chaque ombilic 8, 9 et l'axe 10 ombilical dans un plan, dit plan 57 de première orientation, contenant ledit premier axe 52 de rotation propre et non parallèle à l'axe optique 42b de prise de vues, notamment au moins sensiblement perpendiculaire à cet axe optique 42b de prise de vues. Le plan 57 de première orientation est perpendiculaire au deuxième axe 61 de rotation propre et à l'axe Z vertical, c'est-à-dire parallèle au plan X, Y horizontal contenant les axes 51 des rouleaux 46.

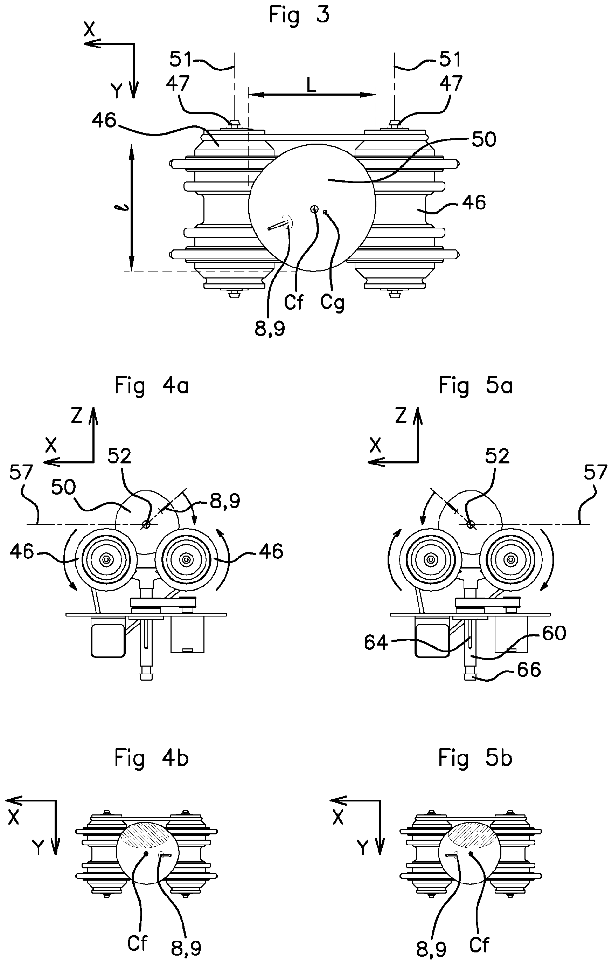

Pour ce faire, au moins une première image, dite image initiale, du fruit est réalisée lors d'une étape 22 d'analyse optique initiale afin de déterminer et d'enregistrer dans une mémoire 23 de l'unité 41 informatique de traitement différents paramètres dimensionnels du fruit et de détecter la présence d'au moins une portion d'un ombilic 8, 9 dans cette image initiale. La figure 3 représente schématiquement ces différents paramètres dimensionnels : la longueur L maximum du fruit selon l'axe X (L = Xmax - Xmin) ; la largeur ℓ maximum du fruit selon l'axe Y (ℓ = Ymax-Ymin) ; la position (XCf, Ycf) du centre Cf géométrique du fruit (par exemple Xcf est le milieu du segment de longueur L et Ycf est le milieu du segment de longueur ℓ) ; la position (XCg, YCg) du centre Cg de gravité du fruit (qui peut être évaluée à partir du barycentre des pixels de l'image initiale) ; les coordonnées (Xt, Yt) d'au moins une portion d'un ombilic éventuellement détecté dans l'image initiale par présence d'une tâche T(II) sombre, ces coordonnées (Xt, Yt) correspondant au centre de la tâche T(II) sombre. Ces paramètres dimensionnels peuvent être déterminés à partir d'une image initiale réalisée dans le domaine infrarouge par la caméra 40b.

Au moins une image initiale, dite image II initiale infrarouge, est réalisée à l'aide de la caméra 40b infrarouge, et la présence d'au moins une portion d'un ombilic dans cette image II initiale infrarouge est détectée sous forme d'une tâche T(II) sombre, par exemple présentant un niveau de gris supérieur à un niveau de gris prédéterminé et de dimension inférieure à celle du fruit mais supérieure à une dimension prédéterminée, ce niveau de gris prédéterminé et cette dimension prédéterminée pouvant être définis expérimentalement en fonction des caractéristiques géométriques des fruits à traiter de façon à fournir une détection fiable de l'ombilic dans l'image. Cette détection est par exemple réalisée à l'aide d'un algorithme de traitement d'image comprenant une convolution et un noyau de convolution.

Après cette étape 22 d'analyse optique initiale, l'unité 41 informatique de traitement commande, lors d'une étape 24 de rotation, la rotation des rouleaux 46 de façon à entraîner le fruit en rotation propre autour du premier axe 52 de rotation propre. Pour ce faire, si une tâche T(II) sombre correspondant à au moins une portion d'un ombilic 8, 9 est détecté dans l'image II initiale infrarouge, l'unité 41 informatique de traitement détermine les positions respectives du centre Cf du fruit et de la tâche T(II) sombre détectée.

L'unité 41 informatique de traitement commande la rotation des rouleaux 46 dans un sens déterminé à partir de l'analyse d'une image initiale en deux dimensions pour éloigner, dans cette image initiale en deux dimensions, l'ombilic détecté du centre Cf du fruit, ce qui permet de minimiser l'amplitude angulaire de déplacement de l'ombilic 8, 9 vers le plan 57 de première orientation. Comme on le voit figures 4a, 4b la tâche T(II) sombre correspondant à l'ombilic 8, 9 étant à droite du centre Cf du fruit, les rouleaux 46 sont entraînés en rotation selon les flèches représentées de façon à entraîner le fruit en rotation propre autour du premier axe 52 de rotation propre dans le sens horaire de la figure 4a. Au contraire, dans la situation représentée figure 5a et 5b, la tâche T(II) sombre correspondant à l'ombilic 8, 9 étant à gauche du centre Cf du fruit, les rouleaux 46 sont entraînés en rotation selon les flèches représentées de façon à entraîner le fruit en rotation propre autour du premier axe 52 de rotation propre dans le sens trigonométrique de la figure 5a.

À chaque étape 24 de rotation le fruit est entraîné en rotation propre autour du premier axe 52 de rotation selon une amplitude angulaire θ comprise entre 5° et 45°. Plus particulièrement, le fruit est entraîné selon une amplitude angulaire θ comprise entre 10° et 20°, en particulier de l'ordre de 15° pour des pommes, cette valeur θ d'amplitude angulaire dépendant en particulier de la technique d'analyse optique utilisée pour détecter l'ombilic 8, 9 dans l'image et des dimensions relatives moyennes de la dépression ombilicale de chaque ombilic du fruit. La valeur optimale peut être déterminée expérimentalement. Elle doit être en particulier suffisamment faible pour garantir que le fruit soit effectivement déplacé (sans glissement), et procurer une précision suffisante de déplacement, notamment de telle sorte que l'ombilic soit correctement orienté après la rotation si seulement une portion de cet ombilic est détectée dans l'image initiale. Elle est suffisamment grande pour optimiser la durée de cette étape et de l'ensemble du procédé.

Après avoir effectué cette étape 24 de rotation, l'unité 41 informatique de traitement commande une nouvelle étape 25 d'analyse optique, dite étape 25 d'analyse optique ultérieure, lors de laquelle au moins une image infrarouge, dite image IU infrarouge ultérieure, du fruit est réalisée par la caméra 40b infrarouge et selon le même axe 42b optique de prise de vues que l'image II initiale infrarouge, et cette image infrarouge ultérieur est analysée par analyse optique pour détecter la présence d'une tâche T(IU) sombre correspondant à au moins une portion d'un ombilic 8, 9 dans cette image IU infrarouge ultérieure, de la même façon que la détection d'un ombilic 8, 9 dans l'image II infrarouge initiale. À l'issue de cette étape 25 d'analyse optique ultérieure, si un ombilic 8, 9 est détecté dans l'image infrarouge ultérieure, les coordonnées (Xt, Yt) du centre de la tâche T(IU) sombre correspondant à cet ombilic sont enregistrées dans la mémoire 23 de masse.

Après l'étape 25 d'analyse optique ultérieure, une étape 32 de décision conditionnelle est exécutée par l'unité 41 informatique de traitement. Dans cette étape 32 de décision conditionnelle, un premier test 26 est exécuté par l'unité 41 informatique de traitement pour déterminer si l'image II infrarouge initiale contient une tâche T(II) sombre correspondant à au moins une portion d'un ombilic 8, 9. Si ce premier test 26 détermine qu'au moins une portion d'un ombilic 8, 9 est détectée dans l'image II infrarouge initiale, un deuxième test 27 est ensuite exécuté par l'unité 41 informatique de traitement pour déterminer si l'image IU infrarouge ultérieure contient aussi une tâche T(IU) sombre correspondant à au moins une portion d'un ombilic 8, 9.

Si le deuxième test 27 détermine qu'au moins une portion d'un ombilic 8, 9 est détectée dans l'image IU infrarouge ultérieure, l'unité 41 informatique de traitement remplace lors de l'étape 30 subséquente l'image II infrarouge initiale par l'image IU infrarouge ultérieure, et reprend la première phase 12 d'orientation en réitérant les étapes 24 de rotation, puis 25 d'analyse optique ultérieure, puis 32 de décision conditionnelle, en considérant donc la précédente image infrarouge ultérieure en tant qu'un nouvelle image infrarouge initiale.

Si le deuxième test 27 détermine qu'aucune portion d'ombilic 8, 9 n'est détectée dans l'image IU infrarouge ultérieure, la première phase 12 d'orientation est arrêtée lors de l'étape 31 et le procédé est poursuivi comme décrit ci-après.

Si le premier test 26 détermine qu'aucune portion d'un ombilic 8, 9 n'est détectée dans l'image II infrarouge initiale un deuxième test 28 est ensuite exécuté par l'unité 41 informatique de traitement pour déterminer si l'image IU infrarouge ultérieure contient aussi une tâche T(IU) sombre correspondant à au moins une portion d'un ombilic 8, 9.

Si le deuxième test 28 détermine qu'au moins une portion d'un ombilic 8, 9 est détectée dans l'image IU infrarouge ultérieure, la première phase 12 d'orientation est arrêtée lors de l'étape 31 et le procédé est poursuivi comme décrit ci-après.

Si le deuxième test 28 détermine qu'aucune portion d'un ombilic 8, 9 n'est détectée dans l'image IU infrarouge ultérieure, un troisième test 29 est exécuté pour déterminer si l'amplitude angulaire θt totale de la rotation du fruit résultant de l' (des différentes) étape(s) 24 de rotation effectuée(s) lors de la première phase 12 d'orientation est ou non supérieure ou égale à une amplitude angulaire, dite amplitude θmax de rotation maximum, prédéterminée comprise entre 180° et 360° -notamment de l'ordre de 270°-. Dans l'affirmative, la première phase 12 d'orientation est arrêtée lors de l'étape 31 et le procédé est poursuivi comme décrit ci-après. Dans la négative, l'unité 41 informatique de traitement remplace lors de l'étape 30 subséquente l'image II infrarouge initiale par l'image IU infrarouge ultérieure, et reprend la première phase 12 d'orientation en réitérant les étapes 24 de rotation, puis 25 d'analyse optique ultérieure, puis 32 de décision conditionnelle, en considérant donc la précédente image infrarouge ultérieure en tant qu'un nouvelle image infrarouge initiale.

À l'issue de cette première phase 12 d'orientation avec rotation propre du fruit autour du premier axe 52 de rotation, l'axe 10 ombilical du fruit se trouve orienté au moins sensiblement dans le plan 57 de première orientation avec une très grande fiabilité. Les différents essais réalisés avec différents fruits de différentes formes et de différents calibres ont démontré qu'en effet ce résultat est atteint quasiment systématiquement, en tout cas de façon suffisamment fiable pour pouvoir envisager son exploitation à l'échelle industrielle.

Après cette première phase 12 d'orientation, le procédé est poursuivi par une deuxième phase 33 d'orientation avec rotation propre du fruit autour du deuxième axe 61 de rotation qui est perpendiculaire au plan 57 de première orientation. Pour ce faire, l'unité 41 informatique de traitement identifie lors de l'étape 13, la dernière image DI infrarouge qui a été réalisée et dans laquelle au moins une portion d'un ombilic 8, 9 a été détectée par analyse optique. Cette dernière image DI infrarouge contenant au moins une portion d'ombilic peut être une image infrarouge initiale ou une image infrarouge ultérieure.

Lors de l'étape 14 subséquente, l'unité 41 informatique de traitement détermine la position du centre Cf du fruit dans cette dernière image DI infrarouge et calcule la valeur d'un angle, dit azimut y, formé entre le premier axe 52 de rotation et l'axe 10 ombilical déterminé lors de cette étape 14 comme étant l'axe passant par le centre de la tâche sombre correspondant à l'ombilic 8, 9 détecté et le centre Cf du fruit.

Lors de l'étape 15 subséquente, l'unité 41 informatique de traitement commande une rotation propre du fruit autour du deuxième axe 61 de rotation selon une amplitude angulaire déterminée par la valeur y calculée d'azimut de façon à orienter l'axe 10 ombilical au moins sensiblement parallèlement au premier axe 52 de rotation.

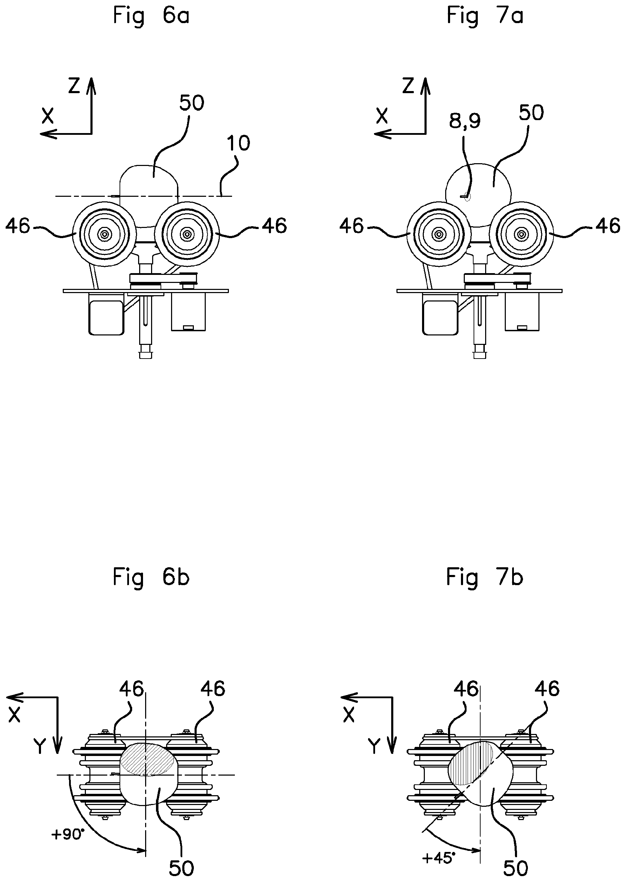

Dans l'exemple représenté sur les figures 6a et 6b, l'axe 10 ombilical est perpendiculaire au premier axe 52 de rotation à l'issue de la première phase 12 de rotation. L'azimut γ de l'axe 10 ombilical est donc de 90°. Lors de l'étape 15 de rotation autour du deuxième axe 61 de rotation, le fruit est donc entraîné en rotation propre par la tige 60 élévatrice selon une amplitude angulaire de 90° autour du deuxième axe 61 de rotation. Dans l'exemple représenté sur les figures 7a et 7b, l'axe 10 ombilical forme un angle de l'ordre de 45° avec le premier axe 52 de rotation à l'issue de la première phase 12 de rotation. L'azimut γ de l'axe 10 ombilical est donc de 45°. Lors de l'étape 15 de rotation autour du deuxième axe 61 de rotation, le fruit est donc entraîné en rotation propre par la tige 60 élévatrice selon une amplitude angulaire de 45° autour du deuxième axe 61 de rotation.

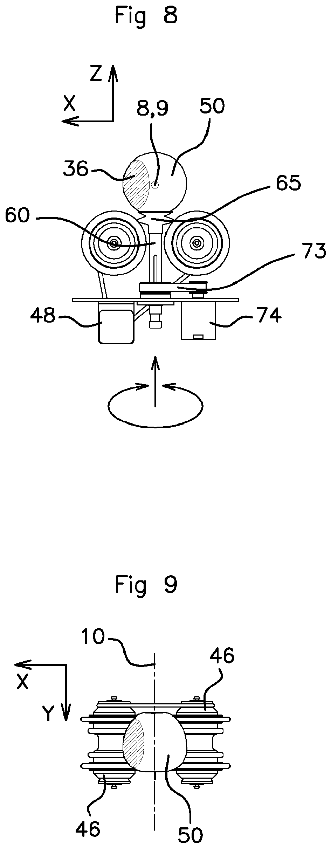

À l'issue de la deuxième phase 33 d'orientation par rotation autour du deuxième axe 61 de rotation, l'axe 10 ombilical est dans le plan 57 de première orientation et est parallèle au premier axe 52 de rotation, c'est-à-dire aux axes 51 de rotation des rouleaux 46 comme représenté sur les figures 8 et 9.

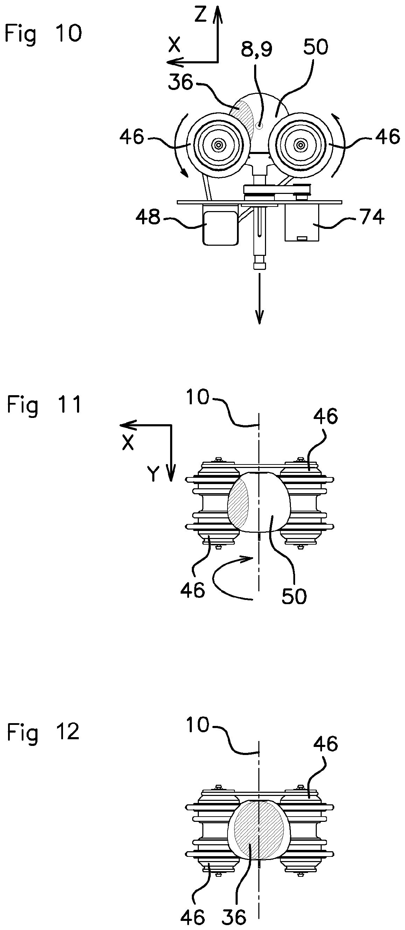

Après la deuxième phase 33 d'orientation, l'unité 41 informatique de traitement exécute une troisième phase 34 d'orientation permettant de placer la portion 36 la plus colorée du fruit, c'est-à-dire présentant une coloration maximum, vers le haut. Pour ce faire, lors de l'étape 16, le fruit est entraîné continûment par les rouleaux 46 en rotation propre autour du premier axe 52 de rotation, selon une amplitude angulaire au moins égale à 360°. Simultanément, une analyse optique du fruit est réalisée par la caméra 40a dans le domaine visible et les images des différentes portions de surface du fruit sont enregistrées au cours de la rotation autour du premier axe 52 de rotation. Ce faisant, l'unité 41 informatique de traitement détermine l'image ainsi réalisée qui correspond à la portion 36 la plus colorée du fruit, ainsi que sa position angulaire autour du premier axe 52 de rotation. Lors de l'étape 17 subséquente, l'unité 41 informatique de traitement commande la rotation des rouleaux 46 de telle sorte que la portion 36 la plus colorée du fruit soit orientée vers le haut, c'est-à-dire vers la caméra 40a, comme représenté sur la figure 12.

Lorsque le fruit est un fruit pédonculé tel qu'une pomme, l'unité 41 informatique de traitement exécute une quatrième phase 37 d'orientation permettant d'orienter le pédoncule 38 du fruit dans un sens prédéterminé et une position angulaire prédéterminée par rapport au premier axe 52 de rotation, de sorte que tous les fruits ainsi orientés présentent tous le pédoncule 38 orienté dans le même sens.

L'unité 41 informatique de traitement exécute tout d'abord une étape 18 d'analyse optique morphologique permettant de détecter la position du pédoncule 38 du fruit. Pour ce faire, l'unité 41 informatique de traitement analyse une image réalisée après la deuxième phase 33 d'orientation dans le domaine visible par la caméra 40a comme représenté figure 16 dans l'exemple d'une pomme pour déterminer :
- la position d'un plan, dit plan 58 équatorial, perpendiculaire à l'axe 10 ombilical et présentant le plus grand diamètre du fruit correspondant à la plus grande valeur de la longueur L selon l'axe X dans cette image, et
- la position d'un plan, dit plan 59 central, perpendiculaire à l'axe 10 ombilical et passant par le centre Cf du fruit.

En effet, dans le cas d'une pomme, le plan 58 équatorial est plus proche du pédoncule 38 que le plan 59 central. Il va de soi que d'autres critères d'analyse morphologique peuvent être utilisés en fonction de la morphologie générale des fruits, pour détecter la position du pédoncule.

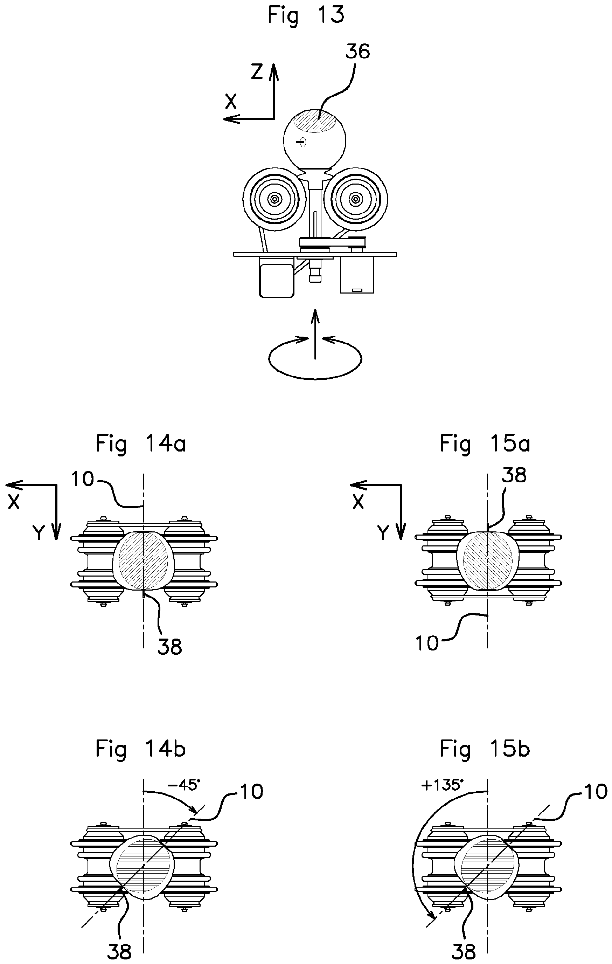

Cette analyse optique morphologique permet donc de déterminer la position du pédoncule 38 sur l'axe 10 ombilical. Lors de l'étape 19 subséquente, l'unité 41 informatique de traitement entraîne le fruit en rotation propre autour du deuxième axe 61 de rotation selon un sens prédéterminé et une amplitude angulaire prédéterminée pour placer le pédoncule 38 dans une position angulaire prédéterminée par rapport au premier axe 52 de rotation, par exemple à 45° comme représenté sur les figures 14b et 15b. Ainsi, selon la position du pédoncule 38 déterminée lors de l'étape 18 d'analyse optique morphologique, le fruit est entraîné en rotation propre soit de 45° dans le sens horaire (exemple des figures 14a et 14b), soit de 135° dans le sens trigonométrique (exemple des figures 15a et 15b).

À l'issue de cette quatrième phase 37 d'orientation, le fruit présente une orientation prédéterminée avec l'axe 10 ombilical dans le plan 57 de première orientation, inclinée à 45° par rapport au premier axe 52 de rotation, avec le pédoncule 38 situé toujours du même côté et la portion 36 colorée vers le haut.

Le dispositif d'emballage selon l'invention représenté sur les figures 19 et 20 comprend un bâti 79 portant une pluralité de dispositifs d'orientation juxtaposés de chaque côté d'un convoyeur 77 à mains 78 basculantes permettant de décharger les fruits 50 d'un côté ou de l'autre du convoyeur 77 sélectivement sur les rouleaux 46 de l'un des supports 44 de ces dispositifs d'orientation. Une brosse 83 rotative permet de freiner la chute des fruits. Le dispositif d'emballage présente, dans l'exemple représenté, deux postes d'emballage, un de chaque côté du convoyeur 77, symétriques l'un de l'autre par rapport à un plan vertical longitudinal médian du convoyeur 77, chaque poste d'emballage comprenant en particulier un robot 80 de manutention et un convoyeur 82 transportant des emballages 81 alvéolés vides sous le robot 80 de manutention.

Sur la figure 19, seul l'un des postes d'emballage est représenté. Ce poste d'emballage comprend dans l'exemple huit supports 44 de huit dispositifs d'orientation juxtaposés le long du convoyeur 77. Dans l'exemple représenté, le robot 80 de manutention comprend un bras vertical 84 portant une main 85 de préhension d'un fruit 50 à son extrémité libre inférieure, ce bras 84 portant un vérin vertical permettant de déplacer la main 85 de préhension verticalement et un actionneur de commande de son actionnement, et étant portés par un portique adapté pour pouvoir déplacer le bras 84 en translations horizontales par rapport au bâti 79 selon deux directions orthogonales horizontales. Chaque caméra 40 réalise des images des fruits de deux dispositifs d'orientation juxtaposés et chaque source 71 de lumière infrarouge est claire au moins deux dispositifs d'orientation juxtaposés.

Lors de l'étape 20 d'emballage, le fruit 50 peut donc être saisi par le robot 80 de manutention pour pouvoir être placé dans un emballage 81 alvéolé (cagettes, plateaux...) avec une orientation optimale prédéterminée. Pour ce faire, l'unité 41 informatique de traitement transmet à une unité de pilotage du robot de manutention les coordonnées du fruit et celles de la position d'une alvéole destinée à recevoir ce fruit, dans un emballage 81 à remplir placé par un convoyeur 82 sous le robot 80 de manutention. L'unité de pilotage du robot 80 génère la trajectoire optimale à effectuer et commande le robot 80 pour le déplacement du fruit. Une fois le fruit déposé dans l'alvéole, l'unité de pilotage du robot confirme que le déplacement de ce fruit dans l'emballage 81 a été réalisé à l'unité 41 informatique de traitement.

Un procédé d'orientation selon l'invention est mis en œuvre par l'unité 41 informatique de traitement qui est programmée à cet effet par un programme d'ordinateur selon l'invention pour exécuter les fonctions techniques mentionnées ci-dessus. Pour ce faire, toute technologie de programmation et/ou langage de programmation informatique peuvent être envisagés (par exemple C, C++, C#,...). De même, l'unité de pilotage du robot de manutention peut être formée de tout automate programmable.

L'invention peut faire l'objet de nombreuses variantes de réalisation par rapport au mode de réalisation représenté sur les figures et décrit ci-dessus. Le dispositif d'analyse optique peut comporter des caméras 40a, 40b aptes à réaliser des prises de vues de différentes caractéristiques, notamment choisies parmi des prises de vues en lumière visible, des prises de vues en lumière visible filtrée, des prises de vues dans le domaine des infrarouges et des prises de vues dans le domaine des ultraviolets. L'invention s'applique à tout fruit ombiliqué. En outre, les images réalisées par les caméras pour l'analyse optique peuvent être des photographies ou des vidéos, l'analyse optique effectuée par l'unité 41 de traitement informatique pouvant être effectuée non seulement sur des photographies mais également sur des vidéos ou des parties de vidéos. D'autres dispositifs et mécanismes d'entraînement en rotation propre au moins selon les deux axes 52, 61 orthogonaux l'un à l'autre peuvent être prévus en lieu et place des rouleaux 46 et de la tige 60 élévatrice. En outre, des sources de lumière appropriées peuvent être également prévues pour éclairer le fruit afin d'améliorer la qualité des images réalisées et la précision d'analyse optique, notamment une source de lumière visible et une source de lumière infrarouge. Les différentes étapes d'un procédé selon l'invention peuvent faire l'objet de nombreuses variantes, et des étapes intercalaires peuvent être prévues entre les étapes successives susmentionnées, dès lors que ces étapes intercalaires n'empêchent pas le fonctionnement du procédé selon l'invention, c'est-à-dire l'exécution de chaque étape de décision conditionnelle et/ou l'orientation appropriée du fruit.

L'invention permet en particulier de réaliser un emballage automatique robotisé de fruits dans des emballages alvéolés, notamment en fin d'une ligne de calibrage de fruits, les fruits étant tous orientés de la même façon, la face la plus colorée vers le dessus, chaque ombilic et chaque pédoncule éventuel étant orienté dans la même direction. Elle s'applique cependant également dans d'autres applications pour lesquelles les mêmes problèmes se posent.

## Revendications

1. Procédé d'orientation d'un fruit ombiliqué dans lequel :
- lors d'une première phase (12) d'orientation le fruit est supporté et entraîné en rotation propre autour d'un premier axe (52) de rotation,
- lors d'une deuxième phase (33) d'orientation ultérieure le fruit est supporté et entraîné en rotation propre autour d'un deuxième axe (61) de rotation orthogonal au premier axe (52) de rotation,
- une analyse optique d'une surface supérieure du fruit est réalisée au moins pendant au moins une partie de la première phase (12) d'orientation à l'aide d'au moins une caméra (40a, 40b) disposée au-dessus du fruit réalisant des images de ladite surface supérieure du fruit, ces images étant transmises à une unité (41) de traitement d'images adaptée pour analyser ces images et produire des résultats d'analyse optique dépendant de l'orientation du fruit,
- la rotation du fruit autour de chacun des deux axes (52, 61) de rotation est commandée en fonction au moins desdits résultats d'analyse optique du fruit, **caractérisé en ce que** :
- la première phase (12) d'orientation comprend les étapes suivantes :
∘ une étape (22) d'analyse optique initiale lors de laquelle :
▪ au moins une image, dite image (II) initiale, du fruit est réalisée selon un axe (42b) optique de prise de vues non parallèle au premier axe (52) de rotation,
▪ chaque image (II) initiale est analysée par analyse optique, la présence d'au moins une portion d'un ombilic (8, 9) étant détectée dans chaque image (II) initiale,
∘ puis une étape (24) de rotation lors de laquelle le fruit est entraîné en rotation propre autour du premier axe (52) de rotation selon une amplitude angulaire comprise entre 5° et 45°,
∘ puis une étape (25) d'analyse optique ultérieure lors de laquelle :
▪ au moins une image, dite image (IU) ultérieure, du fruit est réalisée selon le même axe (42b) optique de prise de vues que l'image (II) initiale,
▪ chaque image (IU) ultérieure est analysée par analyse optique, la présence d'au moins une portion d'un ombilic (8, 9) étant détectée dans chaque image (IU) ultérieure,
- l'unité (41) de traitement exécute une étape (32) de décision conditionnelle selon laquelle :
si une première condition est satisfaite par les résultats d'analyse optique de chaque image (II) initiale et de chaque image (IU) ultérieure, la première phase (12) d'orientation est arrêtée et le procédé est poursuivi par la deuxième phase (33), ladite première condition étant satisfaite si au moins une portion d'un ombilic (8, 9) est détectée dans au moins une image (II) initiale et n'est plus détectée dans chaque image (IU) ultérieure,
si ladite première condition n'est pas satisfaite, les étapes (24) de rotation et (25) d'analyse optique ultérieure de la première phase (12) d'orientation sont réitérées, puis l'étape (32) de décision conditionnelle est réitérée par l'unité (41) de traitement en considérant l'image (IU) ultérieure réalisée avant réitération des étapes (24) de rotation et (25) d'analyse optique ultérieure en tant qu'image initiale,
la réalisation d'au moins deux images successives, ladite au moins une image (II) initiale et ladite au moins une image (IU) ultérieure, séparées d'une rotation du fruit autour du premier axe de rotation (52) et l'étape de décision conditionnelle (32) permettant d'orienter chaque ombilic (8,9) du fruit dans un plan prédéterminé (57), dit plan de première orientation, contenant le premier axe de rotation (52) et non parallèle, notamment au moins sensiblement perpendiculaire, à l'axe optique (42b) de prise de vues de ladite au moins une caméra (40a, 40b) réalisant les images initiale (II) et ultérieure (IU) .

2. Procédé selon la revendication 1 **caractérisé en ce que** selon l'étape (32) de décision conditionnelle :
- si au moins une portion d'un ombilic (8, 9) est détectée dans au moins une image (II) initiale et dans au moins une image (IU) ultérieure, les étapes de rotation et d'analyse optique ultérieure de la première phase d'orientation sont réitérées,
- si au moins une portion d'un ombilic (8, 9) n'est détectée ni dans chaque image (II) initiale ni dans chaque image (IU) ultérieure, les étapes (24) de rotation et (25) d'analyse optique ultérieure de la première phase (12) d'orientation sont réitérées, tant que l'amplitude angulaire totale de la rotation du fruit résultant des différentes étapes (24) de rotation effectuées lors de la première phase (12) d'orientation est inférieure à une amplitude angulaire, dite amplitude de rotation maximum, prédéterminée comprise entre 180° et 360° -notamment de l'ordre de 270 °-,
- si au moins une portion d'un ombilic (8, 9) n'est détectée ni dans chaque image (II) initiale ni dans chaque image (IU) ultérieure, les étapes (24) de rotation et (25) d'analyse optique ultérieure de la première phase (12) d'orientation sont réitérées, et si l'amplitude angulaire totale de la rotation du fruit résultant des différentes étapes (24) de rotation précédemment effectuées lors de la première phase (12) d'orientation est supérieure ou égale à ladite amplitude de rotation maximum, la première phase (12) d'orientation est arrêtée et le procédé est poursuivi,
- si au moins une portion d'un ombilic (8, 9) n'est pas détectée dans chaque image (II) initiale mais est détectée dans au moins une image (IU) ultérieure, les étapes (24) de rotation et (25) d'analyse optique ultérieure de la première phase (12) d'orientation sont réitérées, puis l'étape (32) de décision conditionnelle est réitérée par l'unité (41) de traitement en considérant l'image (IU) ultérieure réalisée avant réitération des étapes de rotation et d'analyse optique ultérieure en tant qu'image initiale.

3. Procédé selon l'une des revendications 1 à 2 **caractérisé en ce que** ledit axe (42b) optique de prise de vues est au moins sensiblement orthogonal au premier axe (52) de rotation.

4. Procédé selon l'une des revendications 1 à 3 **caractérisé en ce que** le premier axe (52) de rotation est contenu dans un plan horizontal et le deuxième axe (61) de rotation est vertical.

5. Procédé selon l'une des revendications 1 à 4 **caractérisé en ce que** ladite étape (22) d'analyse optique initiale comprend une détection d'un centre (Cf) du fruit dans au moins une image (II) initiale, et si au moins une portion d'un ombilic (8, 9) est détectée dans au moins une image (II) initiale, lors de l'étape (24) de rotation subséquente le fruit est entraîné en rotation dans un sens déterminé par les positions respectives du centre (Cf) du fruit et de l'ombilic (8, 9) détectés, et choisi pour minimiser l'amplitude angulaire de déplacement de l'ombilic (8, 9) vers un plan, dit plan (57) de première orientation, contenant le premier axe (52) de rotation et non parallèle à l'axe (42b) optique de prise de vues.

6. Procédé selon l'une des revendications 1 à 5 **caractérisé en ce que** lors de chaque étape (22, 25) d'analyse optique, au moins une image, dite image infrarouge, est réalisée à l'aide d'au moins une caméra (40b) infrarouge, et la présence d'au moins une portion d'un ombilic (8, 9) dans chaque image infrarouge sous forme d'une tâche présentant un niveau de gris supérieur à un niveau de gris prédéterminé et de dimension inférieure à celle du fruit mais supérieure à une dimension prédéterminée.

7. Procédé selon l'une des revendications 1 à 6 **caractérisé en ce que** le deuxième axe (61) de rotation est perpendiculaire à un plan, dit plan (57) de première orientation, contenant le premier axe (52) de rotation et non parallèle à l'axe (42b) optique de prise de vues.

8. Procédé selon l'une des revendications 1 à 7 **caractérisé en ce que** à la fin de la première phase (12) d'orientation l'unité (41) de traitement :
- identifie la dernière image réalisée dans laquelle au moins une portion d'un ombilic (8, 9) est détectée par analyse optique, détermine la position d'un centre (Cf) du fruit dans cette dernière image, et calcule la valeur d'un angle, dit azimut (y), formé entre le premier axe (52) de rotation et un axe, dit axe (10) ombilical, passant par l'ombilic (8, 9) et le centre (Cf) du fruit détectés,
- puis commande une rotation du fruit autour du deuxième axe (61) de rotation lors de la deuxième phase (33) d'orientation selon une amplitude angulaire déterminée par la valeur (γ) calculée d'azimut de façon à orienter l'axe (10) ombilical selon une orientation prédéterminée par rapport au premier axe (52) de rotation.

9. Procédé selon l'une des revendications 1 à 8 **caractérisé en ce qu'**il comprend, après la deuxième phase (33) d'orientation, une phase (34) ultérieure d'orientation lors de laquelle :
- le fruit est supporté et entraîné en rotation sur une amplitude angulaire de rotation d'au moins 360° autour du premier axe (52) de rotation,
- une analyse optique d'une surface supérieure du fruit est réalisée pour détecter une portion de cette surface supérieure, dite portion (36) la plus colorée, présentant une coloration maximum,
- la rotation du fruit est interrompue de façon à placer ladite portion (36) la plus colorée sur le dessus.

10. Procédé selon l'une des revendications 1 à 9 d'orientation d'un fruit pédonculé **caractérisé en ce qu'**il comprend, après la deuxième phase (33) d'orientation, une étape (18) d'analyse optique morphologique adaptée pour permettre une détection de la position d'un pédoncule (38) du fruit.

11. Procédé selon la revendication 10, **caractérisé en ce qu'**il comprend, après l'étape (18) d'analyse optique morphologique, une étape (19) ultérieure de rotation lors de laquelle le fruit est entraîné en rotation autour du deuxième axe de rotation selon une amplitude angulaire déterminée pour placer le pédoncule (38) dans une position angulaire prédéterminée par rapport au premier axe (52) de rotation.

12. Dispositif d'orientation d'un fruit ombiliqué comprenant :
- un premier support (46) de fruit adapté pour supporter un fruit et pour l'entraîner en rotation propre autour d'un premier axe (52) de rotation,
- un deuxième support (60) de fruit adapté pour supporter un fruit et pour l'entraîner en rotation propre autour d'un deuxième axe (61) de rotation orthogonal au premier axe (52) de rotation,
- un dispositif (40a, 40b, 41) d'analyse optique d'une surface (39) supérieure du fruit comprenant au moins une caméra (40a, 40b) disposée au-dessus du fruit pour pouvoir réaliser des images de ladite surface (39) supérieure du fruit,
- une unité (41) de traitement programmable adapté pour :
∘ analyser les images et produire des résultats d'analyse optique dépendant de l'orientation du fruit,
∘ commander la rotation du fruit autour de chacun des deux axes (52, 61) de rotation en fonction aux moins desdits résultats d'analyse optique du fruit,
**caractérisé en ce que** ladite unité (41) de traitement programmable est programmée pour mettre en œuvre un procédé d'orientation selon l'une des revendications 1 à 11.

13. Procédé d'emballage de fruits ombiliqués dans des emballages alvéolés dans lequel chaque fruit est placé dans une alvéole d'emballage selon une orientation prédéterminée **caractérisé en ce qu'**il comprend un procédé d'orientation de chaque fruit selon l'une des revendications 1 à 11.

14. Dispositif d'emballage de fruits ombiliqués comprenant des dispositifs d'orientation des fruits et au moins un robot (80) de manutention des fruits adapté pour placer chaque fruit (50) dans une alvéole d'un emballage alvéolé selon une orientation prédéterminée **caractérisée en ce qu'**il comprend au moins un dispositif d'orientation selon la revendication 12.

15. Programme d'ordinateur comprenant des instructions de code de programme informatique **caractérisé en ce qu'**il comprend des moyens de programmation lisibles par une unité (41) de traitement programmable et adaptés pour, une fois exécutés par ladite unité (41) de traitement programmable, exécuter un procédé d'orientation selon l'une des revendications 1 à 11 avec ladite unité (41) de traitement programmable et avec un dispositif d'orientation de chaque fruit adapté pour supporter et entraîner chaque fruit en rotation propre autour dudit premier axe (52) de rotation et autour dudit deuxième axe (61) de rotation.

16. Programme d'ordinateur comprenant des instructions de code de programme informatique **caractérisé en ce qu'**il comprend des moyens de programmation lisibles par une unité (41) de traitement programmable et adaptés pour, une fois exécutés par ladite unité (41) de traitement programmable, exécuter un procédé d'emballage selon la revendication 13 avec ladite unité de traitement programmable et avec un dispositif d'orientation de chaque fruit adapté pour supporter et entraîner chaque fruit en rotation propre autour dudit premier axe (52) de rotation et autour dudit deuxième axe (61) de rotation.

## Patentansprüche

1. Verfahren zur Ausrichtung einer Nabelfrucht, bei dem:
- die Frucht bei einer ersten Ausrichtungsphase (12) getragen und in Eigenrotation um eine erste Rotationsachse (52) angetrieben wird,
- die Frucht bei einer späteren zweiten Ausrichtungsphase (33) getragen und in Eigenrotation um eine zweite Rotationsachse (61), orthogonal zur ersten Rotationsachse (52), angetrieben wird,
- eine optische Analyse einer oberen Oberfläche der Frucht mindestens während mindestens eines Teils der ersten Ausrichtungsphase (12) mithilfe mindestens einer Kamera (40a, 40b), die oberhalb der Frucht angeordnet ist, realisiert wird, welche Bilder der oberen Oberfläche der Frucht realisiert, wobei diese Bilder zu einer Bildverarbeitungseinheit (41) übertragen werden, die ausgeführt ist, um diese Bilder zu analysieren, und optische Analyseergebnisse zu erzeugen, die von der Ausrichtung der Frucht abhängen,
- die Rotation der Frucht um jede der beiden Rotationsachsen (52, 61) in Abhängigkeit von mindestens den optischen Analyseergebnissen der Frucht gesteuert wird,
**dadurch gekennzeichnet, dass**:
- die erste Ausrichtungsphase (12) die folgenden Schritte umfasst:
∘ einen anfänglichen optischen Analyseschritt (22), bei dem:
• mindestens ein Bild, anfängliches Bild (II) genannt, der Frucht entlang einer optischen Aufnahmeachse (42b), die nicht parallel zur ersten Rotationsachse (52) ist, realisiert wird,
• jedes anfängliche Bild (II) durch optische Analyse analysiert wird, wobei die Präsenz mindestens eines Abschnitts eines Nabels (8, 9) in jedem anfänglichen Bild (II) detektiert wird,
∘ danach einen Rotationsschritt (24), bei dem die Frucht in Eigenrotation um die erste Rotationsachse (52) gemäß einer Winkelamplitude angetrieben wird, die zwischen 5° und 45° enthalten ist,
∘ danach einen Schritt (25) zur späteren optischen Analyse, bei der:
• mindestens ein Bild, späteres Bild (IU) genannt, der Frucht entlang derselben optischen Aufnahmeachse (42b) wie das anfängliche Bild (II), realisiert wird,
• jedes spätere Bild (IU) durch optische Analyse analysiert wird, wobei die Präsenz mindestens eines Abschnitts eines Nabels (8, 9) in jedem späteren Bild (IU) detektiert wird,
- die Verarbeitungseinheit (41) einen Schritt (32) einer bedingten Entscheidung ausführt, bei dem:
falls eine erste Bedingung durch die optischen Analyseergebnisse jedes anfänglichen Bildes (II) und jedes späteren Bildes (IU) erfüllt ist, die erste Ausrichtungsphase (12) angehalten wird, und das Verfahren mit der zweiten Phase (33) fortgesetzt wird, wobei die erste Bedingung erfüllt ist, falls mindestens ein Abschnitt eines Nabels (8, 9) in mindestens einem anfänglichen Bild (II) detektiert wird, und in jedem späteren Bild (IU) nicht mehr detektiert wird,
falls die erste Bedingung nicht erfüllt wird, die Schritte (24) einer Rotation und (25) einer späteren optischen Analyse der ersten Ausrichtungsphase (12) wiederholt werden, danach der Schritt (32) einer bedingten Entscheidung durch die Verarbeitungseinheit (41) unter der Annahme wiederholt wird, dass das spätere Bild (IU) vor der Wiederholung der Schritte (24) einer Rotation und (25) einer späteren optischen Analyse als anfängliches Bild realisiert wurde,
wobei es die Realisation von mindestens zwei aufeinanderfolgenden Bildern, das mindestens eine anfängliche Bild (II) und das mindestens eine spätere Bild (IU), die durch eine Rotation der Frucht um die erste Rotationsachse (52) getrennt sind, und der Schritt einer bedingten Entscheidung (32) ermöglichen, jeden Nabel (8, 9) der Frucht in einer vorbestimmten Ebene (57), erste Ausrichtungsebene genannt, die die erste und nicht parallele Rotationsachse (52), insbesondere im Wesentlichen senkrecht zu der optischen Aufnahmeachse (42b) der mindestens einen Kamera (40a, 40b), die das ursprüngliche (II) und spätere (IU) Bild realisiert, enthält, auszurichten.

2. Verfahren nach Anspruch 1, **dadurch gekennzeichnet, dass** gemäß dem Schritt (32) einer bedingten Entscheidung:
- falls mindestens ein Abschnitt eines Nabels (8, 9) in mindestens einem anfänglichen Bild (II) und in mindestens einem späteren Bild (IU) detektiert wird, die Schritte einer Rotation und einer späteren optischen Analyse der ersten Ausrichtungsphase wiederholt werden,
- falls mindestens ein Abschnitt eines Nabels (8, 9) weder in jedem anfänglichen Bild (II), noch in jedem späteren Bild (IU) detektiert wird, die Schritte (24) einer Rotation und (25) einer späteren optischen Analyse der ersten Ausrichtungsphase (12) wiederholt werden, solange die gesamte Winkelamplitude der Rotation der Frucht, die aus den verschiedenen, in der ersten Ausrichtungsphase (12) durchgeführten, Rotationsschritte (24) resultiert, kleiner als eine vorbestimmte Winkelamplitude, maximale Rotationsamplitude genannt, ist, die zwischen 180° und 360° enthalten - insbesondere in der Größenordnung von 270° - ist,
- falls mindestens ein Abschnitt eines Nabels (8, 9) weder in jedem anfänglichen Bild (II), noch in jedem späteren Bild (IU) detektiert wird, die Schritte (24) einer Rotation und (25) einer späteren optischen Analyse der ersten Ausrichtungsphase (12) wiederholt werden, und falls die gesamte Winkelamplitude der Rotation der Frucht, die aus den verschiedenen, zuvor in der ersten Ausrichtungsphase (12) durchgeführten Rotationsschritte (24) resultiert, größer oder gleich der maximalen Rotationsamplitude ist, die erste Ausrichtungsphase (12) angehalten, und das Verfahren fortgesetzt wird,
- falls mindestens ein Abschnitt eines Nabels (8, 9) weder in jedem anfänglichen Bild (II), noch in jedem späteren Bild (IU) detektiert wird, die Schritte (24) einer Rotation und (25) einer späteren optischen Analyse der ersten Ausrichtungsphase (12) wiederholt werden, danach der Schritt (32) einer bedingten Entscheidung durch die Verarbeitungseinheit (41) unter der Annahme wiederholt wird, dass das spätere Bild (IU) vor der Wiederholung der Schritte einer Rotation und einer späteren optischen Analyse als anfängliches Bild realisiert wurde.

3. Verfahren nach einem der Ansprüche 1 bis 2, **dadurch gekennzeichnet, dass** die optische Aufnahmeachse (42b) mindestens im Wesentlichen orthogonal zur ersten Rotationsachse (52) ist.

4. Verfahren nach einem der Ansprüche 1 bis 3, **dadurch gekennzeichnet, dass** die erste Rotationsachse (52) in einer horizontalen Ebene enthalten ist und die zweite Rotationsachse (61) vertikal ist.

5. Verfahren nach einem der Ansprüche 1 bis 4, **dadurch gekennzeichnet, dass** der anfängliche optische Analyseschritt (22) eine Detektion einer Mitte (Cf) der Frucht in mindestens einem anfänglichen Bild (II) umfasst, und falls mindestens ein Abschnitt eines Nabels (8, 9) in mindestens einem anfänglichen Bild (II) detektiert wird, die Frucht beim nachfolgenden Rotationsschritt (24) in einer Richtung in Rotation angetrieben wird, die durch die jeweiligen detektierten Positionen der Mitte (Cf) der Frucht und des Nabels (8, 9) bestimmt wird, und ausgewählt wird, um die Verschiebungswinkelamplitude des Nabels (8, 9) in eine Ebene, erste Ausrichtungsebene (57) genannt, die die erste Rotationsachse (52) enthält, und nicht parallel zur optischen Aufnahmeachse (42b) ist, zu minimieren.

6. Verfahren nach einem der Ansprüche 1 bis 5, **dadurch gekennzeichnet, dass** bei jedem optischen Analyseschritt (22, 25) mindestens ein Bild, Infrarotbild genannt, mithilfe mindestens einer Infrarotkamera (40b) realisiert wird, und die Präsenz mindestens eines Abschnitts eines Nabels (8, 9) in jedem Infrarotbild in Form eines Flecks, der eine höhere Graustufe als eine vorbestimmte Graustufe und in einer Größe, die kleiner ist als jene der Frucht, jedoch größer als eine vorbestimmte Größe, aufweist.

7. Verfahren nach einem der Ansprüche 1 bis 6, **dadurch gekennzeichnet, dass** die zweite Rotationsachse (61) senkrecht zu einer Ebene, erste Ausrichtungsebene (57) genannt, ist, die die erste Rotationsachse (52) enthält und nicht parallel zur optischen Aufnahmeachse (42b) ist.

8. Verfahren nach einem der Ansprüche 1 bis 7, **dadurch gekennzeichnet, dass** am Ende der ersten Ausrichtungsphase (12) die Verarbeitungseinheit (41):
- das letzte realisierte Bild, in dem mindestens ein Abschnitt eines Nabels (8, 9) durch optische Analyse detektiert wird, identifiziert, die Position einer Mitte (Cf) der Frucht in diesem letzten Bild bestimmt, und den Wert eines Winkels, Azimut (γ) genannt, der zwischen der ersten Rotationsachse (52) und einer Achse, Nabelachse (10) genannt, die durch den detektierten Nabel (8, 9) und die Mitte (Cf) der Frucht verläuft, berechnet,
- danach eine Rotation der Frucht um die zweite Rotationsachse (61) bei der zweiten Ausrichtungsphase (33) gemäß einer durch den berechneten Azimutwert (*γ*) bestimmten Winkelamplitude steuert, um die Nabelachse (10) gemäß einer vorbestimmten Ausrichtung in Bezug auf die erste Rotationsachse (52) auszurichten.

9. Verfahren nach einem der Ansprüche 1 bis 8, **dadurch gekennzeichnet, dass** es nach der zweiten Ausrichtungsphase (33) eine spätere Ausrichtungsphase (34) umfasst, bei der:
- die Frucht getragen und auf einer Rotationswinkelamplitude von mindestens 360° um die erste Rotationsachse (52) in Rotation angetrieben wird,
- eine optische Analyse einer oberen Oberfläche der Frucht realisiert wird, um einen Abschnitt dieser oberen Oberfläche, farbigster Abschnitt (36) genannt, zu detektieren, der eine maximale Färbung aufweist,
- die Rotation der Frucht unterbrochen wird, um den farbigsten Abschnitt (36) an der Oberseite zu platzieren.

10. Verfahren nach einem der Ansprüche 1 bis 9, zur Ausrichtung einer bestielten Frucht, **dadurch gekennzeichnet, dass** es, nach der zweiten Ausrichtungsphase (33) einen Schritt (18) zur geeigneten morphologischen optischen Analyse umfasst, um eine Detektion der Position eines Stiels (38) der Frucht zu ermöglichen.

11. Verfahren nach Anspruch 10, **dadurch gekennzeichnet, dass** es nach dem Schritt (18) einer morphologischen optischen Analyse einen späteren Rotationsschritt (19) umfasst, bei dem die Frucht um die zweite Rotationsachse gemäß einer bestimmten Winkelamplitude in Rotation angetrieben wird, um den Stiel (38) in eine vorbestimmte Winkelposition in Bezug auf die erste Rotationsachse (52) zu platzieren.

12. Vorrichtung zur Ausrichtung einer Nabelfrucht, umfassend:
- einen ersten Fruchtträger (46), der ausgeführt ist, um eine Frucht zu tragen und in Eigenrotation um eine erste Rotationsachse (52), anzutreiben,
- einen zweiten Fruchtträger (60), der ausgeführt ist, um eine Frucht zu tragen und um sie in Eigenrotation um eine zweite Rotationsachse (61), orthogonal zur ersten Rotationsachse (52), anzutreiben,
- eine Vorrichtung (40a, 40b, 41) zur optischen Analyse einer oberen Oberfläche (39) der Frucht, mindestens eine Kamera (40a, 40b) umfassend, die oberhalb der Frucht angeordnet ist, um Bilder der oberen Oberfläche (39) der Frucht realisieren zu können,
- eine programmierbare Verarbeitungseinheit (41) ausgeführt zum:
∘ Analysieren der Bilder und Erzeugen der optischen Analyseergebnisse in Abhängigkeit von der Ausrichtung der Frucht,
∘ Steuern der Rotation der Frucht um jede der beiden Rotationsachsen (52, 61) in Abhängigkeit von mindestens den optischen Analyseergebnissen der Frucht,
**dadurch gekennzeichnet, dass** die programmierbare Verarbeitungseinheit (41) programmiert ist, um ein Verfahren zur Ausrichtung nach einem der Ansprüche 1 bis 11 umzusetzen.

13. Verfahren zum Verpacken von Nabelfrüchten in wabenartigen Verpackungen, wobei jede Frucht gemäß einer vorbestimmten Ausrichtung in eine Verpackungswabe platziert wird, **dadurch gekennzeichnet, dass** es ein Verfahren zur Ausrichtung jeder Frucht nach einem der Ansprüche 1 bis 11 umfasst.

14. Vorrichtung zum Verpacken von Nabelfrüchten, umfassend Vorrichtungen zur Ausrichtung der Früchte und mindestens einen Roboter (80) zur Handhabung der Früchte, ausgeführt, um jede Frucht (50) gemäß einer vorbestimmten Ausrichtung in eine Wabe einer wabenartigen Verpackung zu platzieren, **dadurch gekennzeichnet, dass** sie mindestens eine Vorrichtung zur Ausrichtung nach Anspruch 12 umfasst.

15. Computerprogramm, Computerprogrammcodeanweisungen umfassend, **dadurch gekennzeichnet, dass** es Programmiermittel umfasst, die durch eine programmierbare Verarbeitungseinheit (41) lesbar sind, und ausgeführt sind, um, sobald sie von der programmierbaren Verarbeitungseinheit (41) ausgeführt werden, ein Verfahren zur Ausrichtung nach einem der Ansprüche 1 bis 11 mit der programmierbaren Verarbeitungseinheit (41) und mit einer Vorrichtung zur Ausrichtung jeder Frucht auszuführen, die ausgeführt ist, um jede Frucht zu tragen und in Eigenrotation um die erste Rotationsachse (52) und um die zweite Rotationsachse (61) anzutreiben.

16. Computerprogramm, Computerprogrammcodeanweisungen umfassend, **dadurch gekennzeichnet, dass** es Programmiermittel umfasst, die durch eine programmierbare Verarbeitungseinheit (41) lesbar sind, und ausgeführt sind, um, sobald sie von der programmierbaren Verarbeitungseinheit (41) ausgeführt werden, ein Verfahren zum Verpacken nach Anspruch 13 mit der programmierbaren Verarbeitungseinheit und mit einer Vorrichtung zur Ausrichtung jeder Frucht auszuführen, die ausgeführt ist, um jede Frucht zu tragen und in Eigenrotation um die erste Rotationsachse (52) und um die zweite Rotationsachse (61) anzutreiben.

## Claims

1. A method for orienting an umbilicated fruit wherein:
- during a first orientation phase (12), the fruit is supported and driven in spinning rotation about a first axis (52) of rotation,
- during a second subsequent orientation phase (33), the fruit is supported and driven in spinning rotation about a second axis (61) of rotation orthogonal to the first axis (52) of rotation,
- an optical analysis of an upper surface of the fruit is carried out at least during at least a part of the first orientation phase (12) using at least one camera (40a, 40b) disposed above the fruit producing images of said upper surface of the fruit, these images being transmitted to an image processing unit (41) adapted for analysing these images and producing optical analysis results depending on the orientation of the fruit,
- the rotation of the fruit about each of the two axes (52, 61) of rotation is controlled according to at least said optical analysis results of the fruit, **characterised in that**:
- the first orientation phase (12) comprises the following steps:
-- an initial optical analysis step (22) during which:
--- at least one image, called the initial image (II), of the fruit is produced along an optical shooting axis (42b) not parallel to the first axis (52) of rotation,
--- each initial image (II) is analysed by optical analysis, the presence of at least a portion of an umbilicus (8, 9) being detected in each initial image (II),
-- then a rotation step (24) during which the fruit is driven in spinning rotation about the first axis (52) of rotation at an angular amplitude comprised between 5° and 45°,
-- then a subsequent optical analysis step (25) during which:
--- at least one image, called a subsequent image (UI), of the fruit is produced along the same optical shooting axis (42b) as the initial image (II),
--- each subsequent image (UI) is analysed by optical analysis, the presence of at least a portion of an umbilicus (8, 9) being detected in each subsequent image (UI),
- the processing unit (41) executes a conditional decision step (32) according to which:
if a first condition is met by the results of optical analysis of each initial image (II) and each subsequent image (UI), the first orientation phase (12) is stopped and the method is continued with the second phase (33), said first condition being met if at least a portion of an umbilicus (8, 9) is detected in at least one initial image (II) and is no longer detected in each subsequent image (IU),
if said first condition is not met, the rotation (24) and subsequent optical analysis (25) steps of the first orientation phase (12) are repeated, then the conditional decision step (32) is repeated by the processing unit (41) considering the subsequent image (UI) produced before repeating the rotation (24) and subsequent optical analysis (25) steps as the initial image,
producing at least two successive images, said at least one initial image (II) and said at least one subsequent image (IU), separated by a rotation of the fruit about the first axis of rotation (52) and the conditional decision step (32) allowing to orient each umbilicus (8, 9) of the fruit in a predetermined plane (57), called plane of first orientation, containing the first axis of rotation (52) and not parallel, in particular at least substantially perpendicular to the optical shooting axis (42b) of said at least one camera (40a, 40b) producing the initial (II) and subsequent (IU) images.

2. The method according to claim 1 **characterised in that** according to the conditional decision step (32):
- if at least a portion of an umbilicus (8, 9) is detected in at least one initial image (II) and in at least one subsequent image (IU), the rotation and subsequent optical analysis steps of the first orientation phase are repeated,
- if at least a portion of an umbilicus (8, 9) is detected neither in each initial image (II) nor in each subsequent image (UI), the rotation (24) and optical analysis (25) steps of the first orientation phase (12) are repeated, as long as the total angular amplitude of the rotation of the fruit resulting from the various rotation steps (24) carried out during the first orientation phase (12) is less than an angular amplitude, called the maximum predetermined amplitude of rotation, comprised between 180° and 360° - in particular of the order of 270° -,
- if at least a portion of an umbilicus (8, 9) is detected neither in each initial image (II) nor in each subsequent image (UI), the rotation (24) and optical analysis (25) steps of the first orientation phase (12) are repeated, and if the total angular amplitude of the rotation of the fruit resulting from the various rotation steps (24) previously carried out during the first orientation phase (12) is greater than or equal to said maximum amplitude of rotation, the first orientation phase (12) is stopped and the method is continued,
- if at least a portion of an umbilicus (8, 9) is not detected in each initial image (II) but is detected in at least one subsequent image (UI), the rotation (24) and subsequent optical analysis (25) steps of the first orientation phase (12) are repeated, then the conditional decision step (32) is repeated by the processing unit (41) considering the subsequent image (UI) produced before repeating the rotation and subsequent optical analysis steps as the initial image.

3. The method according to one of claims 1 to 2 **characterised in that** said optical shooting axis (42b) is at least substantially orthogonal to the first axis (52) of rotation.

4. The method according to one of claims 1 to 3 **characterised in that** the first axis (52) of rotation is contained in a horizontal plane and the second axis (61) of rotation is vertical.

5. The method according to one of claims 1 to 4 **characterised in that** said initial optical analysis step (22) comprises detecting a centre (Cf) of the fruit in at least one initial image (II), and if at least a portion of an umbilicus (8, 9) is detected in at least one initial image (II), during the subsequent rotation step (24) the fruit is rotated in a direction determined by the respective detected positions of the centre (Cf) of the fruit and the umbilicus (8, 9), and selected to minimise the angular amplitude of displacement of the umbilicus (8, 9) to a plane, called plane (57) of first orientation, containing the first axis (52) of rotation and not parallel to the optical shooting axis (42b).

6. The method according to one of claims 1 to 5 **characterised in that** during each optical analysis step (22, 25), at least one image, called infrared image, is produced using at least one infrared camera (40b), and the presence of at least a portion of an umbilicus (8, 9) in each infrared image in the shape of a spot having a grey level greater than a predetermined grey level and of dimension less than that of the fruit but greater than a predetermined dimension.

7. The method according to one of claims 1 to 6 **characterised in that** the second axis (61) of rotation is perpendicular to a plane, called plane (57) of first orientation, containing the first axis (52) of rotation and not parallel to the optical shooting axis (42b).

8. The method according to one of claims 1 to 7 **characterised in that** at the end of the first orientation phase (12) the processing unit (41):
- identifies the last produced image wherein at least a portion of an umbilicus (8, 9) is detected by optical analysis, determines the position of a centre (Cf) of the fruit in this last image, and calculates the value of an angle, called azimuth (γ), formed between the first axis (52) of rotation and an axis, called the umbilical axis (10), passing through the umbilicus (8, 9) and the centre (Cf) of the detected fruit,
- then controls a rotation of the fruit about the second axis (61) of rotation during the second orientation phase (33) according to an angular amplitude determined by the calculated azimuth value (γ) so as to orient the umbilical axis (10) in a predetermined orientation relative to the first axis (52) of rotation.

9. The method according to one of claims 1 to 8 **characterised in that** it comprises, after the second orientation phase (33), a subsequent orientation phase (34) during which:
- the fruit is supported and rotated over an angular amplitude of rotation of at least 360° about the first axis (52) of rotation,
- an optical analysis of an upper surface of the fruit is carried out to detect a portion of this upper surface, called the most colourful portion (36), having a maximum colouration,
- the rotation of the fruit is interrupted so as to place said most colourful portion (36) on top.

10. The method according to one of claims 1 to 9 for orienting a pedunculate fruit **characterised in that** it comprises, after the second orientation phase (33), an optical morphological analysis step (18) adapted to allow detection of the position of a peduncle (38) of the fruit.

11. The method according to claim 10, **characterised in that** it comprises, after the optical morphological analysis step (18), a subsequent rotation step (19) during which the fruit is rotated about the second axis of rotation according to a determined angular amplitude to place the peduncle (38) in a predetermined angular position relative to the first axis (52) of rotation.

12. A device for orienting an umbilicated fruit comprising:
- a first fruit support (46) adapted to support a fruit and to drive it in spinning rotation about a first axis (52) of rotation,
- a second fruit support (60) adapted to support a fruit and to drive it in spinning rotation about a second axis (61) of rotation orthogonal to the first axis (52) of rotation,
- a device (40a, 40b, 41) for optical analysis of an upper surface (39) of the fruit comprising at least one camera (40a, 40b) disposed above the fruit in order to be able to produce images of said upper surface (39) of the fruit,
- a programmable processing unit (41) adapted to:
-- analyse the images and produce optical analysis results depending on the orientation of the fruit,
-- control the rotation of the fruit about each of the two axes (52, 61) of rotation according to at least said optical analysis results of the fruit,
**characterised in that** said programmable processing unit (41) is programmed to implement an orientation method according to one of claims 1 to 11.

13. A method for packaging umbilicated fruits in blister packs wherein each fruit is placed in a blister cavity according to a predetermined orientation **characterised in that** it comprises a method for orienting each fruit according to one of claims 1 to 11.

14. A device for packaging umbilicated fruits comprising fruit orientation devices and at least one fruit handling robot (80) adapted to place each fruit (50) in a cavity of a blister pack in a predetermined orientation **characterised in that** it comprises at least one orientation device according to claim 12.

15. A computer program comprising software program code instructions **characterised in that** it comprises programming means readable by a programmable processing unit (41) and adapted for, once executed by said programmable processing unit (41), executing an orientation method according to one of claims 1 to 11 with said programmable processing unit (41) and with a device for orienting each fruit adapted to support and drive each fruit in spinning rotation about said first axis (52) of rotation and about said second axis (61) of rotation.

16. The computer program comprising software program code instructions **characterised in that** it comprises programming means readable by a programmable processing unit (41) and adapted for, once executed by said programmable processing unit (41), executing a packaging method according to claim 13 with said programmable processing unit and with a device for orienting each fruit adapted to support and drive each fruit in spinning rotation about said first axis (52) of rotation and about said second axis (61) of rotation.
